# EUROPEAN PATENT APPLICATION

(11) **EP 3 673 840 A1**
(43) Date of publication of application: **01.07.2020**
(21) Application number: 19217869.7
(22) Date of filing: 19.12.2019
(51) Int. Cl.: A61B 17/22, A61B 17/221, A61M 25/01, A61B 5/00, A61B 34/00, A61B 34/10, A61M 25/00, A61M 25/09

(54) **APPARATUS FOR MANAGING ACUTE ISCHEMIC EVENTS**

(30) Priority: 27.12.2018 US 201862785543 P
(71) Applicant: Neuravi Limited, Galway H91 K5YD (IE)
(72) Inventor: VALE, David, Galway, H91 K5YD (IE); CONNOLLY, Patrick, Galway, H91 K5YD (IE)
(74) Representative: Small, Gary James

(57) **Abstract**

A system is disclosed for use with a clot located in a target vessel for managing one or more acute ischemic events. The system can include a guidewire extending in a longitudinal direction from a proximal end to a distal end, the distal end being configured to control its orientation relative to the clot and the target vessel. The guidewire can include one or more sensors disposed on or adjacent the distal end, the one or more sensors configured for sensing properties of the clot *in vivo* and treating the clot based on the sensed properties.

## Description

### Field

The disclosure relates to acute management of an ischemic event such as a stroke during the hyperacute timeframe in order to treat the patient and minimize brain injury. In particular, endovascular medical systems advanceable through vasculature for managing acute ischemic events.

### Background

The World Health Organization estimates that 15,000,000 strokes occur annually. Approximately 87% of all strokes are ischemic - where a blood clot obstructs the flow of blood to an area of the brain, starving it of oxygen and nutrients. Clots may develop and block vessels locally without being released in the form of an embolus-this mechanism is common in the formation of coronary blockages. Acute obstructions may include blood clots, misplaced devices, migrated devices, large emboli and the like. Thromboembolism occurs when part or all of a thrombus breaks away from the blood vessel wall. This clot is then carried in the direction of blood flow. Clots can include a range of morphologies and consistencies. Long strands of softer clot material may tend to lodge at bifurcations or trifurcations, resulting in multiple vessels being simultaneously occluded over significant lengths. Older clot material can also be less compressible than softer fresher clots, and under the action of blood pressure it may distend the compliant vessel in which it is lodged. Clots may also may vary greatly in length, even in any one given area of the anatomy. For example, clots occluding the middle cerebral artery of an ischemic stroke patient may range from just a few millimeters to several centimeters in length.

Of the 15,000,000 ischemic strokes that occur annually, one-third of patients die and another one-third are disabled. Two of the primary factors associated with mortality in these patients are the occlusion location and the time to treatment. Regarding treatment time from symptom onset, treating the occlusion as fast as practical is important to avoid complications that result in vessels deprived of flow as a result of said occlusion. Intravenous (IV) lytics are used for patients presenting up to 4.5 hours after symptom onset. Guidelines recommend administering IV lytics in the 3-4.5 hour window to those patients who meet the ECASS 3 (European Cooperative Acute Stroke Study 3) trial inclusion/exclusion criteria. As for location, large-vessel occlusions, which are present in 46% of unselected acute stroke patients presenting in academic medical centers, are associated with higher stroke severity. Additionally, not all patients may be treated with thrombolytic therapy, and so mechanical thrombectomy is a valuable alternative in patients contraindicated to t-PA (tissue plasminogen activator) or where t-PA treatment was not effective. These more proximal vessels feed a large volume of brain tissue, ergo clinicians use the presenting NIHSS (National Institute of Health Stroke Scale) score as an indicator of large-vessel occlusion.

With this, it is understood that an ischemic stroke may result if the clot lodges in the cerebral vasculature. It is estimated that 87% of stroke cases are acute ischemic stroke (AIS). In the United States alone, roughly 700,000 AIS cases occur every year and this number is expected to increase with an ageing population. Occlusion of these large arteries in ischemic stroke is associated with significant disability and mortality. Revascularization of intracranial artery occlusions is the therapeutic goal in stroke therapy.

Endovascular mechanical revascularization (thrombectomy) is an increasingly used method for intracranial large vessel recanalization in acute stroke. Such devices based on stent-like technology, referred to as "stentrievers" or "stent-retrievers", are currently displacing first generation thrombectomy devices for recanalization in acute ischemic stroke. There are significant challenges associated with designing clot removal devices that can deliver high levels of performance. There are also a number of access challenges that make it difficult to deliver devices. For example, the vasculature in the area in which the clot may be lodged is often fragile and delicate. In particular, neurovascular vessels are more fragile than similarly sized vessels in other parts of the body and are often just sparsely connected to the surrounding soft tissue bed. Excessive tensile forces applied to these vessels could result in perforations and hemorrhage. A pulmonary embolism is a serious medical condition that carries a relatively high mortality. Pulmonary embolism is generally characterized by a blockage in one of the pulmonary arteries in your lungs and often is caused by one or more blood clots that travel to the lungs. The risk of pulmonary embolism in patients with acute ischemic stroke is known. Pulmonary vessels are larger than those of the cerebral vasculature, but are also delicate in nature, particularly more distal vessels.

There are also significant challenges associated with designing clot removal devices that can deliver high levels of performance. First, the vasculature can present a number of access challenges that make it difficult to deliver devices. In cases where access involves navigating the aortic arch (such as coronary or cerebral blockages), the configuration of the arch in some patients makes it difficult to position a guide catheter. These difficult arch configurations are classified as either type 2 or type 3 aortic arches with type 3 arches presenting the most difficulty.

The reason for poor revascularization results using current devices and approaches is multifaceted. Challenges such as the type of clot, nature of the clot, length of the clot, vascular architecture, and patient comorbidities can play key roles. The presence of vessel tortuosity can further exacerbate the difficulty of addressing these challenging occlusions. Tortuosity can make it more difficult not just to access but also to dislodge the clot, possibly due to the line of force applied to the clot by the device and the potential for vessel movement and deformation.

The tortuosity challenge is even more severe in the arteries approaching the brain. For example, the distal end of the internal carotid artery commonly renders it difficult for the device to navigate a vessel segment with a 180° bend, a 90° bend and a 360° bend in quick succession over just a few centimeters of vessel. In the case of pulmonary embolisms, access is through the venous system and then through the right atrium and ventricle of the heart. The right ventricular outflow tract and pulmonary arteries are delicate vessels that can easily be damaged by inflexible or high profile devices. For these reasons, it is desirable that the clot retrieval device be compatible with as low profile and flexible a guide catheter as possible.

In regards to varying types of clots, clots can range as to both morphology and consistency. The variation is due to multiple factors, including but not limited to origin of the clot, clot location, cellular content, non-cellular content, red blood cells, platelets, and white blood cells. Non-cellular content can include factors such as fibrin, platelets, von Willebrand factor (vWF) (i.e. a blood glycoprotein involved in hemostasis), as well as collagen. Other factors can include levels of serum in the clot, calcified deposits, lipids, clot shape, clot size, heterogeneity of distribution of constituents, as well as pathogenesis.

As a result of these varying factors, certain clots can have long strands of softer clot material which may tend to lodge at bifurcations or trifurcations, resulting in multiple vessels being simultaneously occluded over significant lengths. More mature and organized clot material is likely to be less compressible than a softer fresher clot, and under the action of blood pressure it may distend the compliant vessel in which it is lodged. Further, as understood throughout this disclosure, clots that have a high fibrin content (e.g., higher than 40% fibrin content), rather than relatively higher red blood cell (RBC) count where clots can be softer and less stiff, can have a higher coefficient of friction (e.g., be more stiff and/or be connected more strongly to the vessel wall) that renders fibrin-rich and/or platelet rich clots very difficult to dislodge the clot and may require multiple passes by a device to remove or even dislodge the clot from the vasculature. Further, the properties of the clot may be significantly changed by the action of the retrieval device interacting with the clot (e.g., after the device makes a first pass of the clot). In particular, compression of a blood clot can cause dehydration of the clot and result in a dramatic increase in both clot stiffness and coefficient of friction.

While it is possible to assess many of these characteristics in vitro by examining a particular clot removed during mechanical thrombectomy procedures, in vitro tests have several clear limitations. Foremost, they are time consuming and often destructive to the sample. Samples may have to undergo extensive processing in order to make them amenable to in vitro analysis. Significantly for some pathologies, such as acute ischemic stroke, time to treatment is a critical factor for patient outcomes and in vitro tests may not be a practical way to determine the best treatment for the patient because of the time involved with sampling and analysis.

The solution of this disclosure resolves these and other issues of the art to improve management of acute ischemic events.

### Summary

Disclosed herein are various exemplary devices, systems, and methods of the present disclosure that can address the above needs. In some embodiments, a system is disclosed for use with a clot located in a target vessel for managing one or more acute ischemic events. The system can include a guidewire extending in a longitudinal direction from a proximal end to a distal end, the distal end being configured to control its orientation relative to the clot and the target vessel. The guidewire can include one or more sensors disposed on or adjacent the distal end, the one or more sensors configured for sensing properties of the clot *in vivo* and treating the clot based on the sensed properties.

In some embodiments, the distal end of the guidewire is configured to prevent injury to an inner wall of the target vessel.

In some embodiments, the one or more sensors are near infrared (NIR) sensors disposed on an outer surface of the guidewire.

In some embodiments, the one or more sensors are impedance sensors disposed on an outer surface of the guidewire.

In some embodiments, the one or more sensors are Raman spectroscopy sensors disposed on an outer surface of the guidewire and operable to transmit and collect certain ranges of the electromagnetic spectrum.

In some embodiments, the one or more sensors are one or more fiberoptic strands or bundles disposed on an outer surface of the guidewire and operable to transmit and collect certain ranges of the electromagnetic spectrum.

In some embodiments, the distal end of the guidewire is an atraumatic clot-circumventing configured distal end, and wherein the one or more sensors are circumferentially disposed about an outer surface of the guidewire.

In some embodiments, the distal end of the guidewire is a flattened distal portion having a planar geometric shape and thickness less than an outer diameter of a remaining non-flattened portion. In some embodiments, at least one of the one or more sensors is disposed on the flattened distal portion for sensing properties of the clot and the other of the one or more sensors is disposed on the remaining non-flattened portion for sensing properties of the vessel wall.

In some embodiments, the flattened distal portion has a paddle geometric shape.

In some embodiments, the distal end of the guidewire is an atraumatic clot-circumventing configured distal end that is conformable in the lateral direction complementary to a contour of the inner wall of the target vessel when passed between the inner wall of the target vessel and the clot. When in a compressed state subject to application of an external mechanical force, a widest width in a lateral direction of the distal end of the guidewire is reduceable.

In some embodiments, the system also includes a microcatheter comprising a proximal end and a distal end, wherein the guidewire is advanceable through the microcatheter. While in a non-compressed state not subject to application of an external mechanical force, the atraumatic clot-circumventing distal end of the guidewire having the widest width in the lateral direction greater than the inner diameter of the lumen (e.g., twice as big, thrice as big, etc.).

In some embodiments, a method for managing one or more acute ischemic events, is disclosed. The method can include delivering a guidewire through a microcatheter to a site of a clot in the vasculature, the guidewire comprising one or more sensors connected to or adjacent a distal portion of the guidewire, the one or more sensors configured to measure properties of the clot in the vasculature; sensing properties of the clot using the one or more sensors at a first location of the site of the clot in the vasculature; and generating an output from the sensed properties, whereby the output relates to at least one of a chemical composition and physical properties of the clot.

In some embodiments, the method includes positioning at least one of the one or more sensors on a flattened distal portion for sensing properties of the clot; and positioning the other of the one or more sensors on the remaining non-flattened portion for sensing properties of the vessel wall.

In some embodiments, the method includes classifying the clot based on the sensed properties and generating a classification.

In some embodiments, the method includes ascertaining one or more physical properties of the clot by measuring different spectral features of the clot based on the output.

In some embodiments, the method includes interpreting information contained in the output to determine in real-time at least one of levels of red blood cell content, white blood cell content, levels of fibrin, a clot size, a clot shape, and a clot location in the vasculature.

In some embodiments, the method includes sensing properties of the clot using the one or more sensors at a second location distal or proximal of the clot and the first location; and generating an output from the second location, whereby the output of the second location relates to at least one of a chemical composition and physical properties of the clot.

In some embodiments, the method includes classifying the clot based on the output and one or more of a clinical exam, a blood test, a computerized tomography (CT) scan, a magnetic resonance imaging (MRI) scan, a carotid ultrasound, a cerebral angiogram, and an echocardiogram.

In some embodiments, the method includes determining criteria of the clot by performing at least one of a CT scan and an MRI scan; and analyzing information from at least one of the CT scan and the MRI scan to determine physical and/or chemical composition of the clot, including levels of red blood cell content, white blood cell content, levels of fibrin, a clot size, a clot shape, and/or a clot location in the vasculature.

In some embodiments, the method includes determining criteria of the clot by determining one or more quantitative indications selected from one or more of white blood cell levels, red blood cell levels, serum levels, fibrin levels, clot size, clot location, clot strength, clot elasticity, rate of clot formation or rate of clot lysis.

In some embodiments, an endovascular medical system is disclosed for use with a clot located in a target vessel for managing one or more acute ischemic events. The system can include a guidewire extending in a longitudinal direction from a proximal end to a distal end, wherein the guidewire comprises a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot.

In some embodiments, the one or more sensors are configured to simultaneously measure temperature of a plurality of locations of the clot and target vessel.

In some embodiments, the sensors are selectively positioned at locations along the distal end to measure regions of the clot.

In some embodiments, the one or more sensors are each separated a predetermined distance at locations along the distal end to measure regions of the clot.

In some embodiments, the distal end of the guidewire comprises an expanded perimeter with an atraumatic clot-circumventing configured distal end in a delivery configuration, and wherein the one or more sensors are disposed about the expanded perimeter.

In some embodiments, the expanded perimeter has an elliptical, curved, or paddle geometric shape.

In some embodiments, the distal end of the guidewire comprises an expanded perimeter defined by a loop with a void therebetween in a delivery configuration, the loop comprising two elongate sections extended distally and joined at the distal end, and wherein the one or more sensors are disposed about the elongate sections.

In some embodiments, the loop and the void have an elliptical, curved, or paddle geometric shape.

In some embodiments, the distal end of the guidewire is conformable in the lateral direction complementary to a contour of the inner wall of the target vessel when passed between the inner wall of the target vessel and the clot; and when in a compressed state subject to application of an external mechanical force, a widest width in a lateral direction of the distal end of the guidewire is reduceable.

In some embodiments, a method is disclosed for managing one or more acute ischemic events. The method can include delivering a guidewire through a microcatheter to a site of a clot in the vasculature, the guidewire extending in a longitudinal direction from a proximal end to a distal end and comprising a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot; sensing properties of the clot using the temperature sensors at a first location distal of the clot, a second location in the clot, and third location proximal of the clot; and generating an output from the sensed properties, whereby the output relates to at least one of a chemical composition and physical properties of the clot.

In some examples, the step of sensing properties with the temperature sensors is done simultaneously at each location.

In some examples, the method can include selectively positioning the sensors at predetermined locations along the distal end to measure regions of the clot.

In some examples, the method can include separating each sensor a predetermined distance at locations along the distal end to measure regions of the clot.

In some examples, the distal end of the guidewire comprises an expanded perimeter with an atraumatic clot-circumventing configured distal end in a delivery configuration, and wherein the one or more sensors are disposed about the expanded perimeter.

In some examples, the method can include classifying the clot based on the sensed properties and generating a classification.

In some embodiments, the method can include selecting one or more devices and/or procedural steps to treat the clot based on the output.

In some examples, the method can include interpreting information contained in the output to determine in real-time at least one of levels of red blood cell content, white blood cell content, platelet content, levels of fibrin, a clot size, a clot shape, clot density, and a clot location in the vasculature.

In some examples, the method can include classifying the clot based on the output and one or more of a clinical exam, a blood test, a computerized tomography (CT) scan, a magnetic resonance imaging (MRI) scan, an ultrasound, a cerebral angiogram, and an echocardiogram.

In some embodiments, a method is disclosed for managing one or more acute ischemic events. The method can include delivering a guidewire through a microcatheter to a site of a clot in the vasculature, the guidewire extending in a longitudinal direction from a proximal end to a distal end and comprising a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot; sensing properties of the clot using the temperature sensors at a plurality of locations in the clot; and generating an output from the sensed properties, whereby the output relates to at least one of a chemical composition and physical properties of the clot.

In some examples, the method can include classifying sensing properties of the clot at a location distal and/or proximal of the clot.

In some embodiments, a method for managing one or more acute ischemic events is disclosed. The method can include determining criteria of a clot; classifying the clot based on the criteria and generating a classification; determining an individualized treatment protocol for the clot based on the classification, the individualized treatment protocol comprising one or more techniques selected from using at least one of aspirating, restoring perfusion using a first reperfusion device, and restoring perfusion using a second reperfusion device; and treating the clot based on the individualized treatment protocol.

In some embodiments, the step of classifying the clot is carried out in vivo, for example, using any of the herein disclosed sensing instrumentalities such as the guidewire with one or more sensors.

In some embodiments, the first reperfusion device is a stent retriever and the second reperfusion device is a pinch retriever.

In some embodiments, the classifying the clot comprises one or more of a clinical exam, a blood test, a computerized tomography (CT) scan, a magnetic resonance imaging (MRI) scan, a carotid ultrasound, a cerebral angiogram, and an echocardiogram.

In some embodiments, the determining criteria of the clot can include performing at least one of a CT scan and an MRI scan; and analyzing information from at least one of the CT scan and the MRI scan to determine physical and/or chemical composition of the clot, including levels of red blood cell content, white blood cell content, levels of fibrin, levels of platelets, level of hydration, a clot size, a clot shape, and/or a clot location in the vasculature.

In some embodiments, the first reperfusion device is a stent retriever configured to remove a clot or portions of a clot that are red blood cell rich and the second reperfusion device is a pinch retriever configured to remove a clot or portions of a clot that are fibrin-rich and/or platelet-rich. In certain embodiments, if the classification demonstrates that the clot is red blood cell rich, then the individualized treatment protocol can include passing the first reperfusion device by, through, or about the clot and then retracting the first perfusion device while engaging the clot in a lumen of the first reperfusion device to restore reperfusion to the vessel.

In some embodiments, the first reperfusion device is a stent retriever configured to remove a clot or portions of a clot that are red blood cell rich and the second reperfusion device is a pinch retriever configured to remove a clot or portions of a clot that are fibrin-rich and/or platelet-rich. In certain embodiments, if the classification demonstrates that the clot is fibrin-rich and/or platelet-rich, then the individualized treatment protocol can include passing the second reperfusion device by, through, or about the clot and then retracting the second perfusion device while pinching the clot to restore reperfusion to the vessel.

In some embodiments, a clot is classified as red blood cell rich if the clot is comprised of 30% or more red blood cell count. In some embodiments, a clot is classified as white blood cell rich if the clot is comprised of 30% or more white blood cell count. In some embodiments, a clot is classified as fibrin rich and/or plateletrich if the clot is comprised of 30% or more fibrin.

In some embodiments, the step of determining criteria of the clot includes interpreting information, by a computing device in operative communication with the clot, about the clot to determine in real-time levels (e.g., instantly or within minutes of undertaking the analysis) of at least one of red blood cell content, white blood cell content, levels of fibrin, levels of platelets, levels of hydration, a clot size, a clot shape, and/or a clot location in the vasculature. In some embodiments, the method can also include receiving, through a graphical user interface of the computing device, the individualized treatment protocol, monitoring, by the computing device, perfusion of the vessel with the clot, and, alerting, by the computing device, in response to perfusion being restored in the vessel.

In some embodiments, the computing device is linked to a database comprising correlation data for interpreting information about the clot and determining the individualized treatment protocol. The database can be remote from the computing device.

In some embodiments, the determining criteria of the clot includes delivering a guidewire to a site of the clot in the vasculature, such as the herein disclosed guidewire with one or more sensors; and taking a first reading of the clot by a using instrumentation for near infrared spectroscopy (NIR) coupled to the guidewire at a first location of the site of the clot in the vasculature, and generating a spectrum from the first reading, whereby the spectrum relates to at least one of a chemical composition and/or physical properties of the clot.

In some embodiments, the step of determining criteria of the clot further includes interpreting information contained in the spectrum to determine in real-time at least one of levels of red blood cell content, white blood cell content, levels of fibrin, levels of platelets, level of hydration, a clot size, a clot shape, and/or a clot location in the vasculature.

In some embodiments, the method can also include taking a second reading of the clot by using the instrumentation for NIR at a second location distal or proximal of the clot and the first location, generating a spectrum from the second reading, whereby the spectrum of the second reading relates to at least one of a chemical composition and/or physical properties of the clot, and interpreting information contained in the spectrum of the first reading and the second reading to instantly determine at least one of levels of red blood cell content, levels of white blood cell content, levels of fibrin, levels of platelets, level of hydration, a clot size, a clot shape, and/or a clot location in the vasculature.

In some embodiments, the determining criteria of the clot includes taking measurements of the clot at its proximal end and its distal end, and generating a spectrum from measurements of the clot at its proximal end and its distal end, whereby the spectrum relates to at least one of a chemical composition and/or physical properties of the clot.

In some embodiments, the step of determining criteria of the clot includes delivering a guidewire to a site of the clot in the vasculature, such as any of the herein disclosed guidewires with one or more sensors, taking a first reading of the clot by a using instrumentation for Raman spectroscopy (and/or a visible region of the spectrum) coupled to the guidewire at the site of the clot in the vasculature, and generating a spectrum from the first reading, whereby the spectrum relates to at least one of a chemical composition and/or physical properties of the clot. In some embodiments, the step of determining criteria of the clot also includes interpreting information contained in the spectrum to determine in real-time levels of red blood cell content, white blood cell content, levels of fibrin, levels of platelets, level of hydration, a clot size, a clot shape, and/or a clot location in the vasculature.

In some embodiments, the method includes taking a second reading of the clot by using the instrumentation for Raman spectroscopy at a second location distal or proximal of the clot and the first location, generating a spectrum from the second reading, whereby the spectrum of the second reading relates to a chemical composition and/or physical properties of the clot, and interpreting information contained in the spectrum of the first reading and the second reading to determine in real-time at least one of levels of red blood cell content, white blood cell content, levels of fibrin, levels of platelets, level of hydration, a clot size, a clot shape, and/or a clot location in the vasculature.

In some embodiments, the step of treating of the clot includes retrieving a portion of the clot. The method can also include analyzing the retrieved clot and/or one or more fragments of the clot, and selecting a clot treatment step based on analyzing the retrieved clot or analyzing accessing and crossing the clot. However, this example is not so limited and the clot analysis of this embodiment is contemplated to include analyzing any material in communication or otherwise connected with the respective reperfusion device (e.g., serum or any other particulate associated with the clot).

In some embodiments, the step of determining criteria of the clot includes determining one or more quantitative indications selected from one or more of white blood cell levels, red blood cell levels, serum levels, fibrin levels, levels of platelets, level of hydration, clot size, clot location, clot strength, clot elasticity, rate of clot formation or rate of clot lysis. The method can include comparing the first and the second clot characteristic quantitative indications to correlation data and determining a selection and/or order of using one or more techniques selected from aspiration, a first reperfusion device, and/or a second reperfusion device.

In some embodiments, a system for treating an ischemic event is disclosed. The system can include a means for providing in vivo analysis information of a clot of the subject having an ischemic event, a means for providing an indication of an individualized treatment protocol for the subject based upon the analysis information, the individualized treatment comprising one or more techniques selected from using aspiration, restoring perfusion using a first reperfusion device, and/or restoring perfusion using a second reperfusion device; and means for treating the clot based on the individualized treatment protocol.

In some embodiments, the first reperfusion device of the system is a stent retriever configured to remove a clot or portions of a clot that are red blood cell rich and the second reperfusion device is a pinch retriever configured to remove a clot or portions of a clot that are fibrin-rich and/or platelet-rich. In some embodiments, if the indication demonstrates that the clot is red blood cell rich, then the individualized treatment protocol includes a means for passing the first reperfusion device by, through, or about the clot and then retracting the first perfusion device while engaging the clot in a lumen of the first reperfusion device to restore reperfusion to the vessel.

In some embodiments, the first reperfusion device is a stent retriever configured to remove a clot or portions of a clot that are red blood cell rich and the second reperfusion device is a pinch retriever configured to remove a clot or portions of a clot that are fibrin-rich and/or platelet-rich. In some embodiments, if the classification demonstrates that the clot is fibrin-rich and/or platelet-rich, then the individualized treatment protocol includes a means for passing the second reperfusion device by, through, or about the clot and then retracting the second perfusion device while pinching the clot to restore reperfusion to the vessel.

In some embodiments, a system for treating an ischemic event is disclosed. The system can include a first reperfusion device for restoring perfusion to an occluded vessel having a clot. The system can include a second reperfusion device for restoring perfusion to the occluded vessel having the clot. The system can include a delivery system for delivering at least one of the first and second reperfusion devices to the clot in the occluded vessel. The system can include a clot analysis system for analyzing the clot of the occluded vessel and determining an individualized treatment protocol. The delivery system can include at least one of a guide catheter, a guidewire, a microcatheter and the like, each of which are operable to delivered through the vasculature to the site of the clot.

To the accomplishment of the foregoing and related ends, certain illustrative aspects are described herein in connection with the following description and the appended drawings. These aspects are indicative, however, of but a few of the various ways in which the principles of the claimed subject matter may be employed and the claimed subject matter is intended to include all such aspects and their equivalents. Other advantages and novel features may become apparent from the following detailed description when considered in conjunction with the drawings.

### Brief Description of the Drawings

The disclosure will be more clearly understood from the following description of some embodiments thereof, given by way of example only with reference to the accompanying drawings in which:
FIG. 1 shows a patient catheterized via femoral access with an example clot retrieval device positioned in a cerebral vessel using the arterial system for its delivery.
FIG. 2 shows certain anatomy of cerebral arteries above the aortic arch leading to the brain.
FIG. 3 shows an isometric view of an example stent retriever device of this disclosure.
FIG. 4 is a perspective view of a pinching retriever device according to an embodiment of this disclosure.
FIG 5 is a schematic overview of an example of this disclosure.
FIG 6 is a schematic overview of one example of a clot analysis approach of this disclosure.
FIG 7 is a schematic overview of an example of clot analysis using non-contrast CT.
FIG 8 is a schematic overview of an example of clot analysis using brain MRI and/or advanced MR
FIG 9 is a schematic overview of an example of clot analysis using contrast enhanced CT.
FIG 10 is a schematic overview of an example of clot analysis using catheter angiography.
FIG 11 is schematic overview of an example of clot analysis using catheter angiography.
FIG. 12 is a system diagram illustrating an operating environment capable of implementing aspects of the present disclosure in accordance with one or more example embodiments.
FIG. 13 is a conventional mechanical thrombectomy system including a guidewire having a conventional distal tip or end undesirably introduced into a perforator vessel after crossing through a clot, occlusion or blockage.
FIG. 14A is a view of a guidewire from FIG. 13 along section A-A.
FIG. 14B is a view of another guidewire from FIG. 13 along section A-A.
FIG. 15 is an illustration of an example guidewire with sensors for detecting properties of a clot.
FIG. 16 is an illustration of an example guidewire with sensors for detecting properties of a clot.
FIG. 17A is an exemplary illustration of an atraumatic clot-circumventing configured distal tip of a guidewire.
FIG. 17B is a cross section view of Fig. 17A taken along section B-B.
FIG. 18 is an exemplary illustration of an atraumatic clot-circumventing configured distal tip of a guidewire.
Fig. 19A depicts a device for sensing temperature of a clot and surrounding vasculature.
Fig. 19B depicts a device for sensing temperature of a clot and surrounding vasculature.
Fig. 20A depicts a device for sensing temperature of a clot and surrounding vasculature.
Fig. 20B depicts a device for sensing temperature of a clot and surrounding vasculature.
Fig. 21A depicts a device for sensing temperature of a clot and surrounding vasculature.
Fig. 21B is a close-up view of one example of a clot for analysis using solutions of this disclosure.
FIG. 22 is a computer architecture diagram showing a general computing system capable of implementing aspects of the present disclosure in accordance with one or more embodiments described herein.
FIG. 23 depicts a schematic overview of a mobile device analyzing a clot or fragments of a clot in vitro recently removed from the patient.
FIG. 24 depicts an overview of histopathologic composition of clots analyzed in this disclosure.
FIG. 25a depicts a graph showing the stress-strain curve of a clot in contracted and uncontracted states identified as having 0% red blood cell count.
FIG. 25b depicts a graph showing the stress-strain curve of for a clot in contracted and uncontracted states identified as having 40% red blood cell count.
FIG. 25c depicts a graph showing the stress-strain curve of for a clot in contracted and uncontracted states identified as having 5% red blood cell count.
FIG. 26 shows a flow diagram depicting an example method of this disclosure for managing one or more acute ischemic events.
FIG. 27 shows a flow diagram depicting an example method of this disclosure for managing one or more acute ischemic events.

### Detailed Description

Some aspects of the present disclosure relate to methods and systems for analyzing and/or classifying acute ischemic events, in vivo and/or in vitro, as well as individualizing a treatment protocol for the particular acute ischemic event. Although example embodiments of the present disclosure are explained in detail herein, it is to be understood that other embodiments are contemplated. Accordingly, it is not intended that the present disclosure be limited in its scope to the details of construction and arrangement of components set forth in the following description or illustrated in the drawings. The present disclosure is capable of other embodiments and of being practiced or carried out in various ways.

As discussed herein, the terms "distal" or "proximal" are used in the following description with respect to a position or direction relative to the treating physician or medical interventionalist. "Distal" or "distally" are a position distant from or in a direction away from the physician or interventionalist. "Proximal" or "proximally" or "proximate" are a position near or in a direction toward the physician or medical interventionist. The terms "occlusion", "clot" or "blockage" are used interchangeably.

As discussed herein, a "subject" may be any applicable human, animal, or other organism, living or dead, or other biological or molecular structure or chemical environment, and may relate to particular components of the subject, for instance specific tissues or fluids of a subject (e.g., human tissue in a particular area of the body of a living subject), which may be in a particular location of the subject, referred to herein as an "area of interest" or a "region of interest."

As discussed herein, a "sensor" may be any device or element of a device that is capable of detecting or measuring or reading or storing or otherwise communicating a physical property of the clot or vasculature or other feature of a subject of this disclosure.

As discussed herein, "treatment protocol" may be any plan to resolve an ischemic event observed in a patient, such as restoring perfusion to an occluded vessel. For example, a treatment protocol can include one or a combination of using aspiration, a stent retriever device, a pinch retriever device, thrombolytic infusion, or any other mechanical, fluid, or other means to restore perfusion to the occluded vessel. The treatment protocol can be individualized based on a number of factors for a particular patient, as discussed more particularly below.

An "ASPECT" score means the Alberta Stroke Program Early computed tomography (CT) score (ASPECTS) that includes a 10-point quantitative topographic CT scan score.

As discussed herein, "mRS" means the modified Rankin Scale (mRS) that is a commonly used scale for measuring the degree of disability or dependence in the daily activities of people who have suffered a stroke or other causes of neurological disability. The mRS scale runs from 0-6, running from perfect health without symptoms to death. An mRS score of 0 is understood as no symptoms being observed. An mRS score of 1 is understood as no significant disability is observed and the patient is able to carry out all usual activities, despite some symptoms. An mRS score of 2 is understood as slight disability and the patient is able to look after own affairs without assistance, but unable to carry out all previous activities. An mRS score of 3 is understood as moderate disability whereby the patient can require some help, but is able to walk unassisted. An mRS score of 4 is understood as moderate severe disability and the patient is unable to attend to own bodily needs without assistance or walk unassisted. An mRS score of 5 is understood as severe disability and the patient requires constant nursing care and attention, bedridden, incontinent. An mRS score of 6 is understood as the patient being deceased.

As discussed herein, the term "computed tomography" or CT means one or more scans that make use of computer-processed combinations of many X-ray measurements taken from different angles to produce cross-sectional (tomographic) images (virtual "slices") of specific areas of a scanned object, allowing the user to see inside the object without cutting. Such CT scans of this disclosure can refer to X-ray CT as well as many other types of CT, such as positron emission tomography (PET) and single-photon emission computed tomography (SPECT).

As discussed herein, modified treatment in cerebral ischemia (mTICI) score categorizes the amount of flow restoration after endovascular revascularization. Specifically, the mTICI score was developed from the original Thrombolysis in Cerebral Infarction (TICI) scale by a consensus group in 2013. The recommendations included a name change to better reflect the increasing use of endovascular therapy for stroke, and simplification of the TICI 2 component to less than half of the target vascular territory (mTICI 2a) or more than half (mTICI 2b). Classification: Grade 0: no perfusion; Grade 1: antegrade reperfusion past the initial occlusion, but limited distal branch filling with little or slow distal reperfusion; Grade 2; Grade 2a: antegrade reperfusion of less than half of the occluded target artery previously ischemic territory (e.g. in one major division of the middle cerebral artery (MCA) and its territory); Grade 2b: antegrade reperfusion of more than half of the previously occluded target artery ischemic territory (e.g. in two major divisions of the MCA and their territories); Grade 3: complete antegrade reperfusion of the previously occluded target artery ischemic territory, with absence of visualized occlusion in all distal branches. Although in most cases excellent rates of mTICI 2b or 3 reperfusion can be achieved using current technology, challenging cases remain in which satisfactory reperfusion is not achieved despite several passes and manipulations of the thrombectomy device in a large percentage of cases (e.g., approximately 20% or more cases). The solution of this disclosure provides systems and methods for all types of cases, including the approximately 20% or more excluded under current approaches.

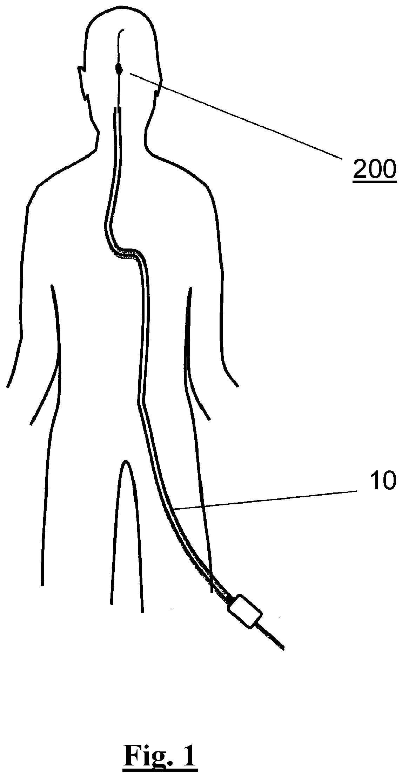

A detailed description of aspects of the present disclosure will now be provided with reference to the accompanying drawings. The drawings form a part hereof and show, by way of illustration, specific embodiments or examples. In referring to the drawings, like numerals represent like elements throughout the several figures. As an example, FIG. 1 depicts a schematic representation of the catheterization of a patient with a clot retrieval device 200 via the femoral artery with a microcatheter 10. Example device 200 can restore blood flow in the neurovascular by removing thrombus in patients experiencing ischemic stroke within a certain period of time from symptom onset (e.g., 8 hours, 12 hours, 24 hours, etc.).

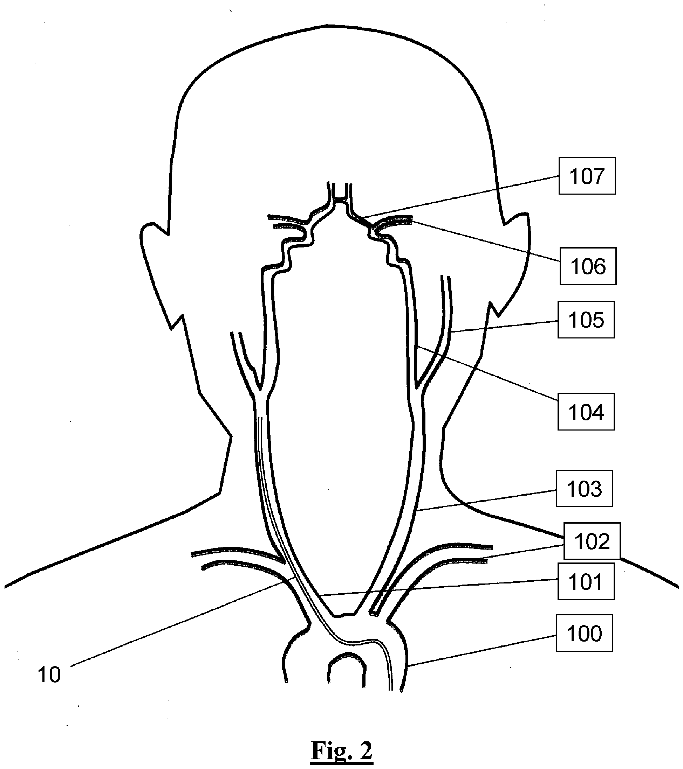

FIG. 2 shows a schematic representation of certain example cerebral vessels. Vessel 100 is the Aorta. Vessel 101 is the brachiocephalic artery. Vessel 102 is the subclavian artery. Vessel 103 is the common carotid artery. Vessel 104 is the internal carotid artery. Vessel 105 is the external carotid artery. Vessel 106 is the middle cerebral artery. Vessel 107 is the anterio-cerebral artery. The microcatheter 10 from FIG. 1 is shown with its distal end in the common carotid artery. In the more detailed drawings of the invention the details of the access site will not be shown but in general access and delivery is in accordance with FIG. 1 and/or FIG. 2. In addition, device 200 can be delivered through the vascular in the wrist (radially) or directly by accessing the carotid artery. Device 200 can be designed for use in the anterior and posterior neurovasculature in vessels such as the internal carotid artery, the M1 and M2 segments of the middle cerebral artery, the vertebral artery, and the basilar arteries. Device 200 can be delivered endovascularly under fluoroscopic guidance in a similar manner to that of other neurovascular clot-retrieval systems.

Once across the site of vessel occlusion, the stent-like element of device 200 is deployed to entrap the clot and allow it to be retrieved, hence restoring blood flow. Device 200 can be a dual-layer stent retriever, with articulating petals, and a distal capture zone for effectively trapping, retaining, and removing various clot types to restore blood flow in patients with AIS secondary to large-vessel occlusion. Device 200 can be available in two lengths, 5x21 mm and 5x33 mm.

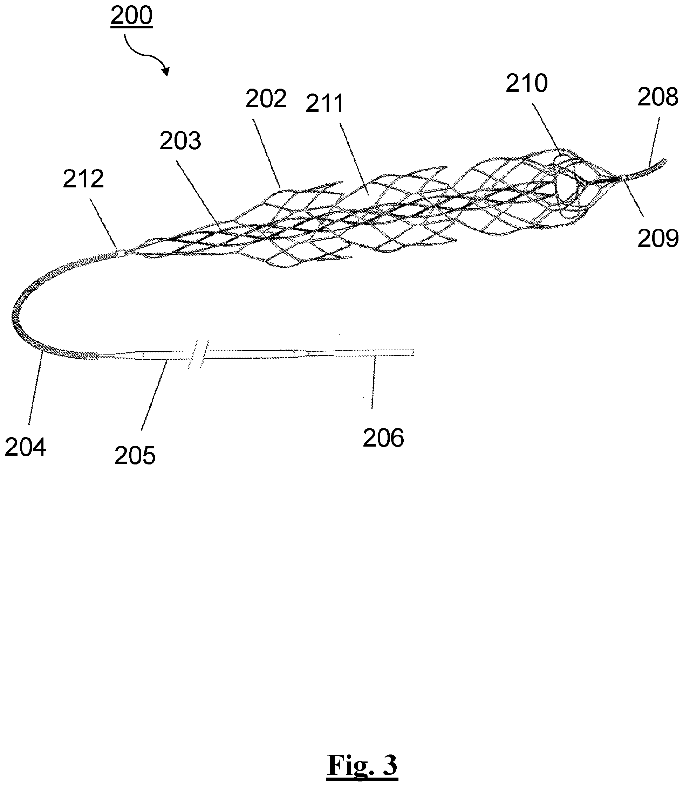

FIG. 3 shows one embodiment of an example stent retriever device 200 of this disclosure. Device 200 can be understood as including features more clearly described in U.S. Pat. Nos. 8,777,976; 8,852,205; 9,402,707; 9,445,829; and 9,642,639, each of which are incorporated by reference in their entirety as if set forth verbatim herein. Device 200 can have an elongate shaft 206. Shaft 206 can have a distal end that extends interior of the artery and a proximal end that extends exterior of the artery. Shaft 206 can also have a clot engaging portion configured at its distal end having an outer expandable member 202 and an inner expandable member 203 to facilitate restoration of blood flow through the clot after device 200 is deployed. Members 202 and 203 can be configured to have a collapsed configuration for delivery and an expanded configuration for clot retrieval, restoration of perfusion, and fragmentation protection in general.

Shaft 206 may be a tapered wire shaft, and may be made of stainless steel, MP35N, Nitinol or other material of a suitably high modulus and tensile strength. Shaft 206 has a coil 204 adjacent its distal end and proximal of the outer member and inner tubular member. The coil may be coated with a low friction material or have a polymeric jacket positioned on the outer surface. Sleeve 205 may be positioned on shaft 206 adjacent coil 204. Sleeve 205 may be polymeric and may be positioned over the tapered section of shaft 206.

The outer member 202 is configured to self-expand upon release from a microcatheter to a diameter larger than that of the inner tubular member 203. Expansion of the outer member 202 causes compression and/or displacement of the clot during expansion for purposes of restoring perfusion to the vessel. A radiopaque coil 208 (which may be platinum or gold or an alloy of same) is positioned over the distal end of member 203 and butts against the distal collar 209 of the outer member 202, where it is connected by an adhesive joint to the collar 209. Inlet openings of outer member 202 can provide the primary movement freedom available to the clot and so the expansion of the outer member 202 urges the clot into the reception space 211 and outer member 202 can have multiple inlet mouths to accept the clot. Optionally expanded distal struts 210 can be included with the inner member 203 and function as an additional three-dimensional filter to prevent the egress of clot or clot fragments.

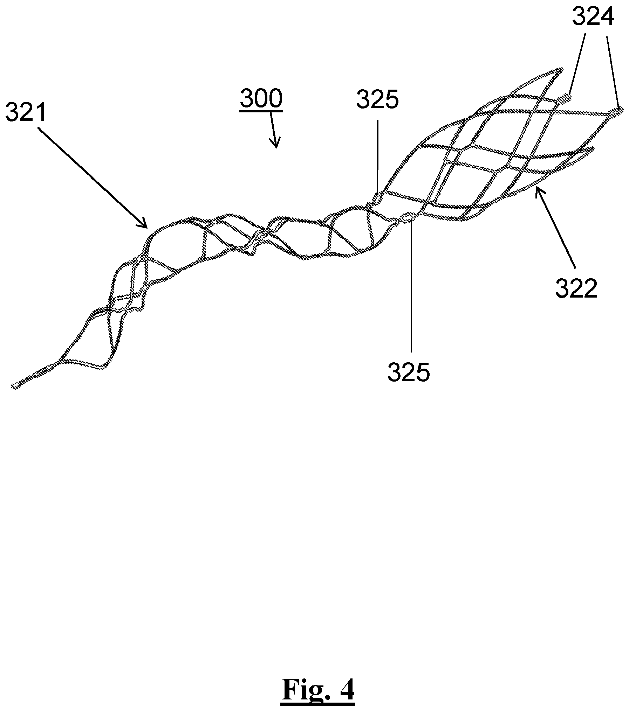

Referring to FIG. 4 there is illustrated an example pinching retriever device 300 according to an embodiment of this disclosure that can be configured for removing fibrin rich and/or platelet trich clots. Device 300 can be understood as including features more clearly described in U.S. App. No. 16/021,505, which is incorporated by reference in its entirety as if set forth verbatim herein. Device 300 can include a proximal pinch section 321, a distal section 322, distal marker coils 324 and radiopaque markers 325. The proximal pinch section 321 can be heat set into a spiral shape having the following features: spiral pitch - 14mm (within a range of 10 - 25 mm); spiral outer diameter - 5mm (within a range of 4.0 to 10 mm); and the spiral typically may form a 360° curve, or range from 180 to 720°. A longitudinal center axis of the distal barrel section 322 may be offset from a center line of the spiral to assist in achieving uniform (low strain) connection between the sections.

Device 300 can have an expandable structure with a constrained delivery configuration, an expanded clot engaging deployed configuration, and an at least partially constrained clot pinching configuration, whereby at least a portion of the expandable structure is configured to engage the clot in the expanded deployed configuration and to pinch clot on movement from the deployed configuration to the clot pinching configuration. The distal end of device 300 can have a spiral section orientated so that it is perpendicular to the proximal face of the barrel section. In this orientation, both the struts connecting the spiral section to the barrel section are equal length and have equivalent levels of strain regardless of the cut pattern orientation on the heat forming mandrel. In other embodiments, the spiral section may be orientated at an angle to the barrel section. Device 300 can include staggering of the radiopaque markers 325 as well as longitudinal staggering of the markers. The proximal end of the pinch section of the device 300 in some cases is attached to a shaft (similar to device 200) using a mechanical locking system.

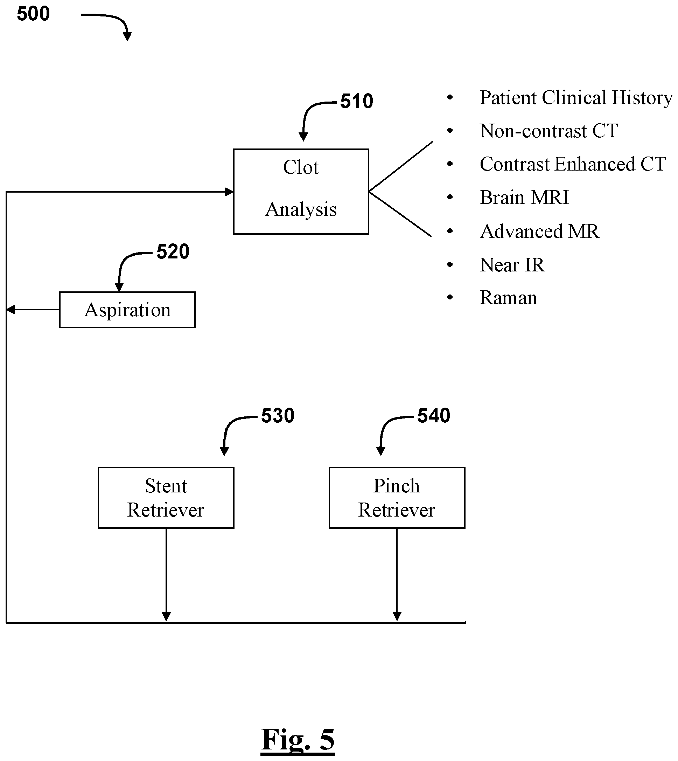

Turning to FIG. 5 is a graphical overview of one embodiment of the method 500. As can be seen, one step of method 500 can include clot analysis 510. Clot analysis 510 can include a variety of some or all of a range of steps, without limitation: considering the patient's clinical history, stroke severity, such as the National Institute of Health Stroke Severity (NIHSS) clinical exam and/or neurological exam. Steps of clot analysis 510 can also include blood tests, non-contrast computerized tomography (CT) scan, including quantitative methods to analyze stroke severity, such as Alberta stroke program early CT score (e.g., ASPECTS), and automatic assessments of ASPECTS using software (e-ASPECTS).

The clinical history of the patient may include factors such as whether the patient is aged between 18 years and 85 years; an mRS score of 0 or 1; angiographic confirmation of an occlusion of an internal carotid artery (ICA) (including T or L occlusions), M1 or M2 MCA, VA, or BA with mTICI flow of 0 - 1; MRI criterion: volume of diffusion restriction visually assessed ≤50 mL.; CT criterion that includes an ASPECTS score of 6 to 10 on baseline CT or CTA-source images, or, volume of significantly lowered CBV ≤50 mL; life expectancy likely less than 6 months; females who were pregnant or breastfeeding; history of severe allergy to contrast medium; known nickel allergy at time of treatment; known current use of cocaine at time of treatment; patient has suffered a stroke in the past 3 months; the patient presents with an NIHSS score <8 or >25 or is physician assessed as being in a clinically relevant uninterrupted coma; the use of warfarin anticoagulation or any Novel Anticoagulant with International Normalized Ratio (INR) >3.0; platelet count <50,000/µL; glucose <50 mg/dL; any known hemorrhagic or coagulation deficiency; unstable renal failure with serum creatinine >3.0 or Glomerular Filtration Rate (GFR) <30; patients who received a direct thrombin inhibitor within the last 48 hours; a partial thromboplastin time (PTT) less than 1.5 times the normal to be eligible; patients with severe hypertension on presentation (SBP > 220 mmHg and/or DBP > 120 mm Hg); cerebral vasculitis; improving neurological status; clinical symptoms suggestive of bilateral stroke or stroke in multiple territories; ongoing seizure due to stroke; evidence of active systemic infection; cancer with metastases; CT or MRI evidence of recent hemorrhage on presentation; baseline CT or MRI showing mass effect or intracranial tumor (except small meningioma); suspicion of aortic dissection, presumed septic embolus, or suspicion of bacterial endocarditis; stenosis, or any occlusion, in a proximal vessel that requires treatment or prevents access to the site of occlusion; evidence of dissection in the extra or intracranial cerebral arteries; and/or occlusions in multiple vascular territories (e.g., bilateral anterior circulation, or anterior/posterior circulation).

Step 510 can also include dual energy CT scanning whereby one normal X-ray and also a second less powerful X-ray are used concurrently to make the images. The two X-rays will generate different spectra using different tube potentials. Step 510 can also include an approach that uses CT scanning as described in U.S. App. No. 16/001,427, which is incorporated by reference in its entirety as if set forth verbatim herein.

Step 510 can also include an MRI and/or advanced MR images of the patient's brain to evaluate the clot. Advanced MR images can include sophisticated magnetic resonance imaging techniques that evaluate freedom of water molecule movement in a selected area, the microvascular integrity and hemodynamic characteristics, and the chemical makeup of the clot. Advanced MR can include perfusion imaging, diffusion-weighted imaging, and MR spectroscopy, as well as magnetic resonance angiography, and/or magnetic resonance venography.

Step 510 can also include advanced MR sequences, such as T1-wieghted imaging, T2-wieghted imaging, diffusion weighted imaging (DWI), proton density imaging, fluid attenuated inversion recovery (FLAIR), Short T1 inversion recovery (STIR), perfusion imaging, apparent diffusion coefficient maps (ADC), gradient echo imaging (GRE), Post-Gadolinium enhancement scans, and/or T2 relaxation time.

Step 510 can also include carotid ultrasound, cerebral angiogram, echocardiogram, intravascular ultrasound (IVUS), and/or optical coherence tomography (OCT).

Step 510 can also include one or more blood tests as well as a non-contrast and/or contrast CT scan of the patient, including the brain area to look at the structures of the brain and evaluate the clot or clots, particularly since no preparation is required for the patient.

Step 510 can also include spectroscopic techniques such as Near Infrared Spectroscopy (NIR) and/or Raman spectroscopy to take readings at one or more locations on the clot and corresponding vasculature to produce a spectrum that relates to the chemical composition and physical properties of the respective occlusion. In this respect, information contained in the spectral bands can be interpreted to provide almost instant analysis of the nature of the material being tested. In certain embodiments, instrumentation associated with the NIR and/or Raman spectroscopy can be included in a microcatheter associated with the delivery system that is delivered to the site of the occlusion in connection with steps 520, 530, and/or 540.

In certain embodiments, in step 510, the occlusion can be scanned in vivo using a catheter having a fiberoptic bundle core connected to a NIR or Raman spectrophotometer. The distal end of the catheter can have a mirror set at 45° on to which light coming from the spectrophotometer via the fiberoptic will be reflected 90° towards the wall of the vessel. Light that is scattered and reflected can be transmitted back to the spectrophotometer via the same mirror and fiberoptic bundle. A spectrum of the transmitted light can be generated, and this information can be used to predict the composition of the material that the light was reflected from. For example, chemical information that corresponds to the bulk composition of the clot and vessel can be deciphered from light absorptions in the near infrared portion of the electromagnetic spectrum and can be used to measure the relative composition of RBC, water, fibrin, or the like within the clot and presence of underlying atheroma in the vessel. Physical information that can be detected in this embodiment can relate to the compactness and organization in the clot resulting from scattering and diffusion of light.

Step 510 can also include analyzing content that may be embedded with a stent retriever after each pass. For example, after a first pass, in certain embodiments the clot, or fragments thereof, can be analyzed in vitro to determine criteria associated therewith. For example, a red blood cell count, a white blood cell count, serum level, fibrin level, or the like can be established to classify the clot. A sample of the clot may then undergo visual or tactile analysis to assist in selection of the proper device used for further procedures.

As a result of analysis stage 510, an indication of clot composition can be provided that advantageously allows classification of the clot in both qualitative and quantitative terms as follows, including the exclusion of presence of a hemorrhagic stroke. Such information can include cellular constituents, extracellular constituents, morphology, organization and distribution of components, permeability, adhesion, water content, resistance to degradation, fibrin crosslink density, fiber diameter, modulus, strain, deformation (e.g., elastic, plastic, viscoelastic), compressibility, and/or fracture behavior. An example table of such indications is provided herein without limitation and other qualitative and/or quantitative indications are contemplated for use with the herein disclosed embodiments:

| **Classification Parameter** | **Range** | **Procedure 520, 530, 540 Indicator** |
|---|---|---|
| Hyperdense middle cerebral artery sign (HMCAS) | Present or absent in relation to contralateral vessel | Present suggestive of aspiration 520 and a stent retriever 530 Absent suggestive of pinch retriever 540 |
| Susceptibility Vessel Sign (SVS) or blooming artefact (BA) | Present or absent in relation to contralateral vessel | Present suggestive of aspiration 520 and a stent retriever 530 Absent suggestive of pinch retriever 540 |
| Catheter angiography, tactile feedback | Subjective force required to pass thrombus | High suggestive of pinch retriever 540 Low suggestive of aspiration 520 and stent retriever 530 |
| Catheter angiography, success of recanalization | Number of attempts, >=1 | >1 suggestive of pinch retriever 540 |
| Clot retrieval, color on gross morphology | White (devoid or RBCs) to dark red (full or RBCs) | Closer to white suggestive of pinch retriever 540. Closer to dark red suggestive of aspiration 520 or stent retriever 530. |
| Clot retrieval, tactile feedback | Soft and friable to Firm and cohesive | Firm clot suggestive of pinch retriever 540. Soft and friable clot suggestive of aspiration 520, stent retriever 530. |

Step 510 can include one or a combination of the foregoing clot analysis embodiments. For example, step 510 can include analyzing criteria of the CT scan and the MRI scan, comparing data analyzed in each to determine levels of red blood cell content, white blood cell content, levels of fibrin, levels of platelets, levels of hydration, a clot size, a clot shape, a clot location in the vasculature, and/or the like. Depending on the classification following step 510, a treatment protocol can be individualized for a patient that dictates whether step 520, 530, and/or 540 is carried out, which advantageously avoids needless use of time from the available timeframe in attempting procedures which are not effective in the circumstances, especially in the context of treatment of acute ischemic stroke. For example, if the red blood cell count and/or the fibrin levels cause the clot to be classified as fibrin-rich and/or platelet-rich, then step 540 may be carried out for a device operable to dislodge and retrieve the fibrin-rich and/or platelet-rich clot. The result of step 510 can also be a decision to either take no action, or to implement the one or more of steps 520, 530, and/or 540.

In some embodiments, step 520 of method 500 can include aspiration of the clot through a catheter and/or a syringe and/or one or more electromechanical pump in accordance with the embodiments of this disclosure, including devices and corresponding systems described in WO2015/0189354A, which is incorporated herein by reference. Step 520 can also be initiated based on information (e.g., classification) from the step of clot analysis 510.

In some embodiments, step 530 can include introducing a stent retriever of this disclosure into the vasculature of the patient for intracranial large vessel recanalization in acute stroke in accordance with the embodiments of this disclosure. Example stent retrievers can include the device 200. Step 530 can also be initiated based on information from the step of clot analysis 510.

Step 540 can include introducing a pinch retriever into the vasculature, which can also be informed by clot analysis 510, including devices and corresponding systems as previously described with respect to device 300. It is understood that the pinch retriever associated with step 540 can be operable to be delivered through the vasculature to the site of the clot to pinch at least a portion of the clot body as its expandable element is at least partially collapsed from a fully expanded configuration. The expandable element of the pincher device can be configured to come into contact with at least a portion of the clot, while maintaining the position of the elongate member steadfast and effecting pinching substructure of the device so as to pinch at least a portion of the clot and retracting the clot retrieval device and the pinched occlusive clot from the patient. Step 540 can also be initiated based on information from the step of clot analysis 510.

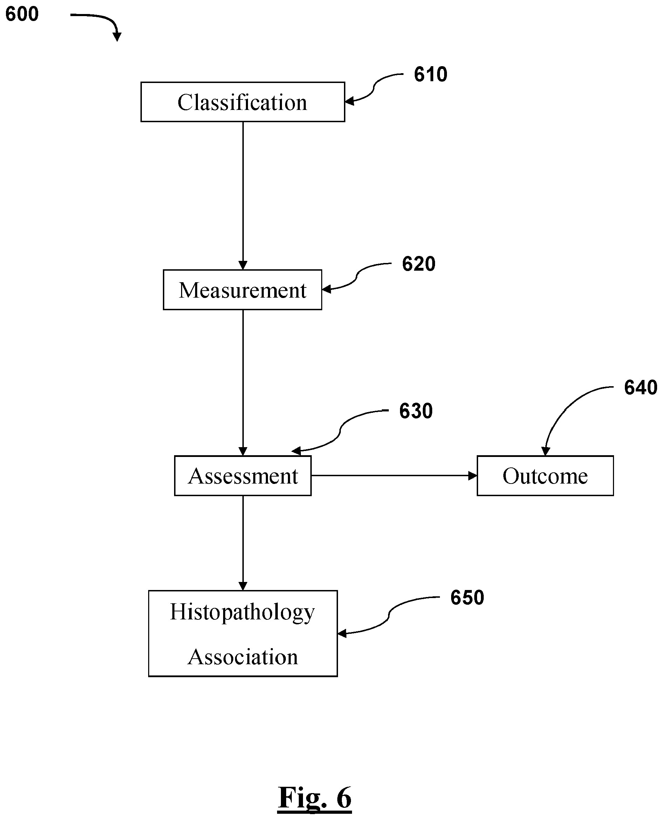

Each of the steps of method 500 can be carried concurrent with clot analysis 510 in a feedback loop so that information from any of the steps of method 500 are executed dependent on information relative to the clot being treated. Clot analysis 510 can be performed by example systems and methods discussed more particularly in FIGs 6-11. However, the embodiments depicted in these figures are by no way limiting and other clot analysis approaches are contemplated as included in this disclosure.

Turning to FIG 6 is a graphical overview of one embodiment of the method 500. As can be seen, one step of method 600 can include classification 610 of the clot present in the vessel. Classification 610 can be carried out by analyzing criteria based on one or more of the embodiments previously described in regards to the clot analysis of method 500. Classification 610 can be selected amongst one or more predetermined classifications. For example, classifications of the clot can include levels or concentration of fibrin, red blood cells, white blood cells, serum, friction, or the like.

Step 620 of method 600 can include taking one or more measurements of the clot, in vivo and/or in vitro, depending on the method employed in classification 610. Once the measurement 620 is taken, the step of assessment 630 can be carried out for the step of histopathology association 650 and/or individualized treatment, device selection for restoring perfusion to the vessel, and corresponding outcome 640.

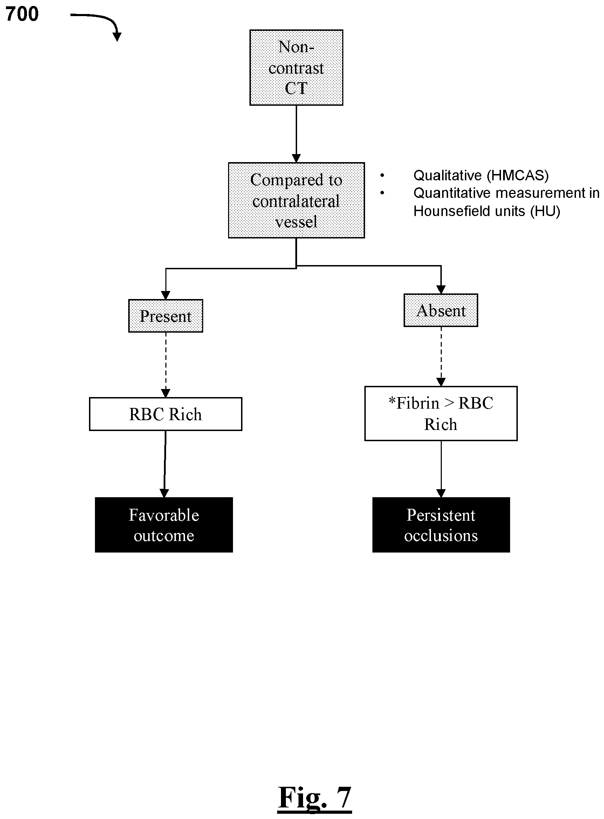

Turning to FIG 7 is a graphical overview of one embodiment of the method 600 of a specific clot analysis embodiment. As can be seen, one step of method 600 can include carrying out non-contrast CT of the clot in the vessel. The non-contrast CT is compared to the contralateral vessel, including qualitatively with respect to hyperdense middle cerebral artery signs (HMCAS) and quantitatively in household units (HU). Based on this analysis of method 700, it can be determined whether present in the clot is a red blood cell (RBC) rich structure or a fibrin-rich and/or platelet-rich structure, since clots may be either RBC rich or fibrin rich and/or plateletrich. If following the analysis in vivo of method 700 it is determined that the clot is RBC rich, then the outcome of thrombectomy using the stent retriever of this disclosure will likely be favorable. Conversely, if following the analysis in vivo of method 700 it is determined that the clot is fibrin rich and/or plateletrich, then the outcome of thrombectomy using the stent retriever of this disclosure will likely be unfavorable and instead, a pinch retriever may be preferable.

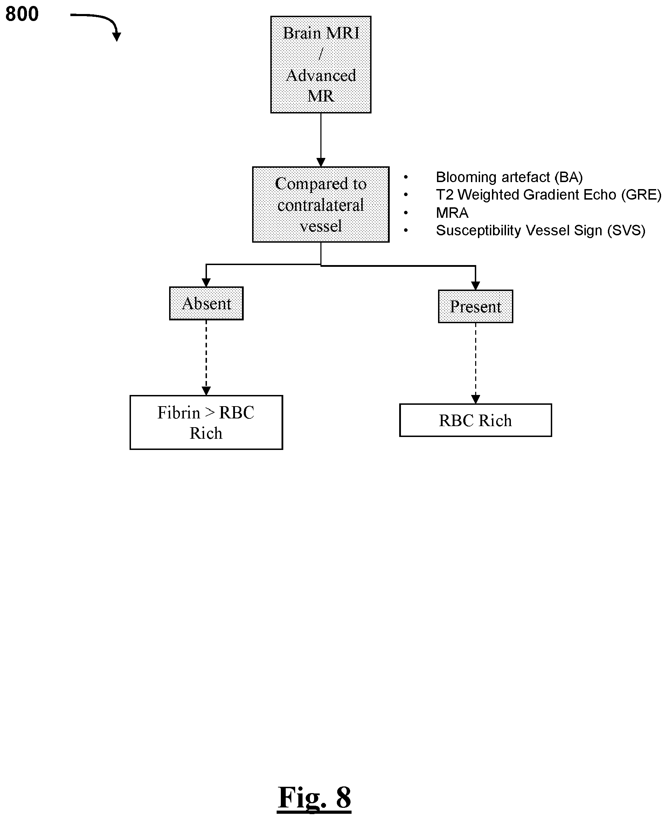

Turning to FIG 8 is a graphical overview of one embodiment of the method 800 of a specific clot analysis embodiment. As can be seen, one step of method 800 can include carrying out a brain MRI and/or advanced MR of the clot in the vessel. The brain MRI and/or advanced MR is compared to the contralateral vessel, including qualitatively with respect to blooming artefact (BA), T2 weighted gradient echo (GRE), MRA, and susceptibility vessel sign. Based on this analysis of method 800, it can be determined whether present in the clot is a red blood cell (RBC) rich structure or a fibrin-rich and/or platelet-rich structure. If following the analysis in vivo of method 800 it is determined that the clot is RBC rich, then the outcome of thrombectomy using the stent retriever of this disclosure will likely be favorable. Conversely, if following the analysis in vivo of method 800 it is determined that the clot is fibrin rich and/or plateletrich, then the outcome of thrombectomy using the stent retriever of this disclosure will likely be unfavorable and instead, a pinch retriever may be preferable.

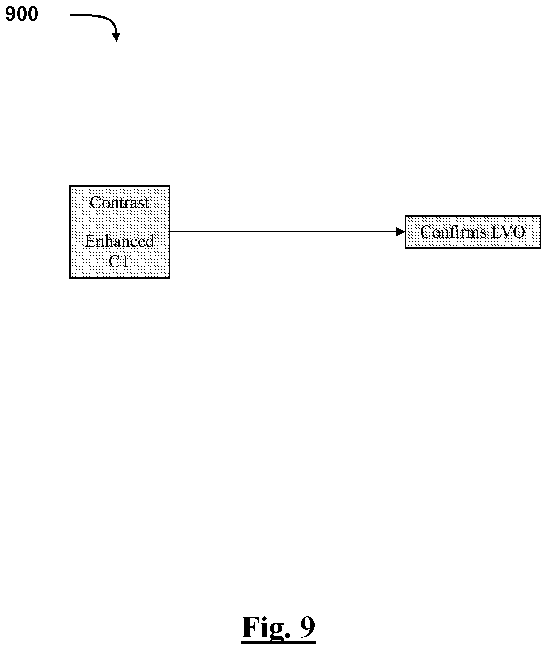

Turning to FIG 9 is a graphical overview of one embodiment of the method 800 of a specific clot analysis embodiment. As can be seen, one step of method 800 can include carrying out contrast enhanced CT scan of the brain whereby depending on the scan, it can be determined that a large vessel occlusion (LVO) is present or not to initiate one or more steps of methodologies of this disclosure.

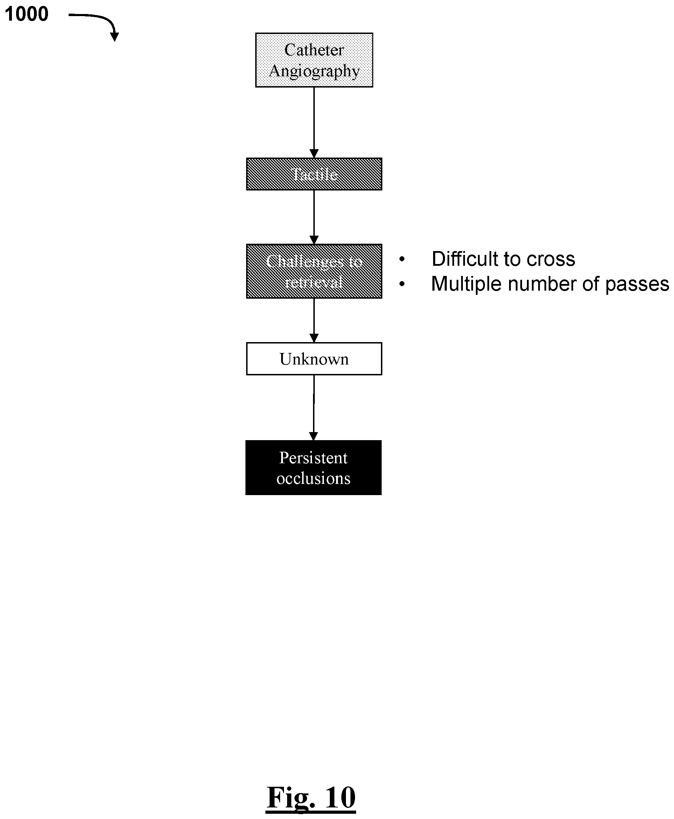

Turning to FIG 10 is a graphical overview of one embodiment of the method 1000 of a specific clot analysis embodiment. As can be seen, one step of method 900 can include catheter angiography to visualize the inside, or lumen, of the occluded vessel. Based on this visualization, method 1000 can determine if the clot is tactile or exhibits other physical properties. As a result, it can be determined whether there will be challenges to retrieval of the clot, including as a result of tortuosity of the respective vessel and visualized characteristics of the clot. For example, if it is determined that tactility of the clot is related to a fibrin rich and/or plateletrich composition, this can dictate that the correct treatment protocol to restore perfusion may be multiple passes of one or more retriever devices (e.g., a stent retriever and/or a pincher retriever). It is also contemplated that one retriever may retrieve portions of the visualized clot whereas the other retriever may retriever the remainder.

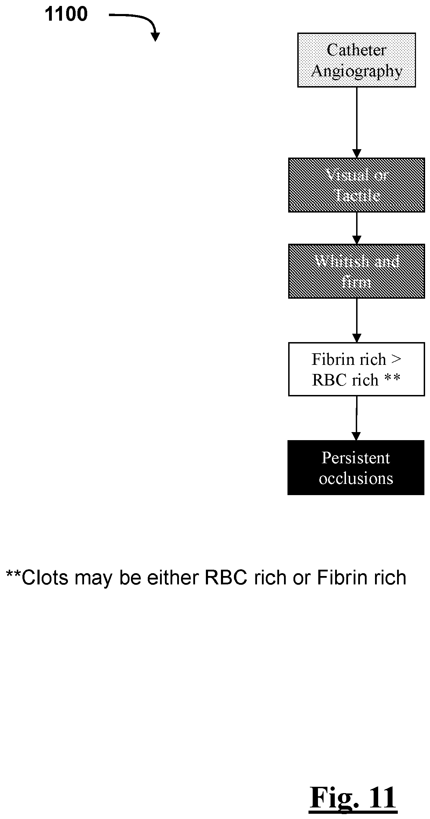

Turning to FIG 11 is a graphical overview of one embodiment of the method 1000 of a specific clot analysis embodiment. As can be seen, one step of method 1100 can include catheter angiography to visualize the inside, or lumen, of the occluded vessel. Based on this visualization, method 1100 can visualize the clot as well as determine if the clot is tactile or exhibits other physical properties. As a result, it can be determined features of the clot, for example, based on color or firmness, and this can be a telltale to the extent of the challenges to retrieval of the clot. For example, in some embodiments, firmness or whitish color visualized of the clot can indicate that the clot should be classified as fibrin rich and/or plateletrich, rather than RBC rich. Depending on this analysis, the proper treatment protocol can be selected to restore perfusion to the vessel.

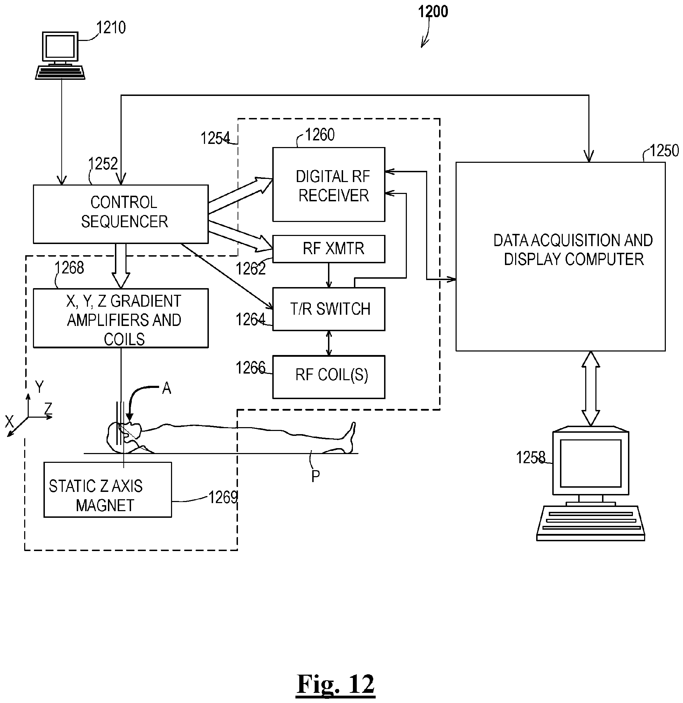

FIG. 12 illustrates an example of one embodiment where a brain MRI is conducted during the clot analysis phases. An MRI system 1200, including a data acquisition and display computer 1250 coupled to an operator console 1210, an MRI real-time control sequencer 1252, and an MRI subsystem 1254. The MRI subsystem 1254 may include XYZ magnetic gradient coils and associated amplifiers 168, a static Z-axis magnet 1269, a digital RF transmitter 1262, a digital RF receiver 1260, a transmit/receive switch 1264, and RF coil(s) 1266. The MRI subsystem 1254 may be controlled in real time by control sequencer 1252 to generate magnetic and radio frequency fields that stimulate magnetic resonance phenomena in a subject P to be imaged, for example to implement magnetic resonance imaging sequences in accordance with various embodiments of the present disclosure. A contrast-enhanced image of an area of interest A of the subject P (which may also be referred to herein as a "region of interest") may be shown on display 1258. The display 1258 may be implemented through a variety of output interfaces, including a monitor, printer, or data storage or even a mobile device.

The area of interest A corresponds to a region of the brain associated with the clot of subject P. The area of interest shown in the example embodiment of FIG. 12 corresponds to a brain region, but it should be appreciated that the area of interest for purposes of implementing various aspects of the disclosure presented herein is not limited to the brain area. It should be recognized and appreciated that the area of interest in various embodiments may encompass various areas of subject P associated with various physiological characteristics.

It should be appreciated that any number and type of computer-based medical imaging systems or components, including various types of commercially available medical imaging systems and components, may be used to practice certain aspects of the present disclosure. Systems as described herein with respect to example embodiments are not intended to be specifically limited to MRI implementations or the particular system shown in FIG. 12. One or more data acquisition or data collection steps as described herein in accordance with one or more embodiments may include acquiring, collecting, receiving, or otherwise obtaining data such as imaging data corresponding to an area of interest. By way of example, data acquisition or collection may include acquiring data via a data acquisition device, receiving data from an on-site or off-site data acquisition device or from another data collection, storage, or processing device. Similarly, data acquisition or data collection devices of a system in accordance with one or more embodiments of the present disclosure may include any device configured to acquire, collect, or otherwise obtain data, or to receive data from a data acquisition device within the system, an independent data acquisition device located on-site or off-site, or another data collection, storage, or processing device.

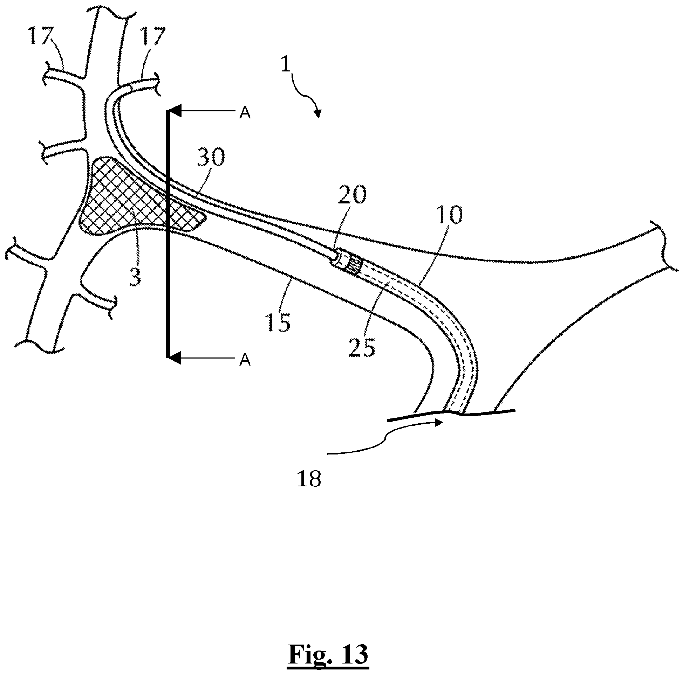

During the thrombectomy procedure or treatment a physician or interventionalist endovascularly introduces a guidewire through the vasculature, typically in an artery located in the groin or by direct access through the carotid artery, as shown in Fig. 13. In particular, when advanced across the occlusion or clot 3 first, the guidewire 30 may undesirably: (i) enter a perforator vessel 17 that branches from a target, main or primary artery 15 potentially causing vessel damage and perforation; and/or (ii) cause injury or damage to the vessel tissue when passed across the clot. To minimize or prevent injury to the target vessel tissue and/or perforator vessels, when the guidewire 30 is advanced across the clot 3 prior to the microcatheter 10, the physician or interventionalist may deliberately change or alter the guidewire 30 by bending its distal end. However, shaping or altering of the distal tip or end of the guidewire 30 in such manner is difficult to control by the physician or interventionalist possibly resulting in the shaped or altered distal end or tip being incapable of passing across or around the clot 3 and/or potentially causing deleterious injury or damage to the target vessel tissue when passed (or attempted to be passed) across or around the clot 3. The guidewire 30 is advanced through the vasculature to a location facing a proximal side of the targeted clot 3, blockage or occlusion. Once the guidewire 30 is properly positioned, a microcatheter 10 with an outer diameter typically less than approximately 1.0 mm, tracks over the guidewire 30 passing through a lumen 25 defined axially through the microcatheter 10.

Guidewires commonly used for these types of procedures are relatively small in diameter so that they may easily pass through a lumen having a relatively small inner diameter defining a lumen 25 extending longitudinally through the microcatheter 10. Conventional guidewire 30 outer diameter sizes range from approximately 0.010" to approximately 0.014", while the inner diameter range of the lumen 25 of the microcatheter 10 is between approximately 0.016" and approximately 0.027". Typically, the outer diameter of the guidewire 30 used is 0.014", while the inner diameter of the microcatheter 10 is 0.021". Smaller size guidewires having an approximately 0.010" outer diameter and microcatheters having an inner diameter lumen of approximately 0.016" are sometimes used, particularly in smaller or more distal vessels.

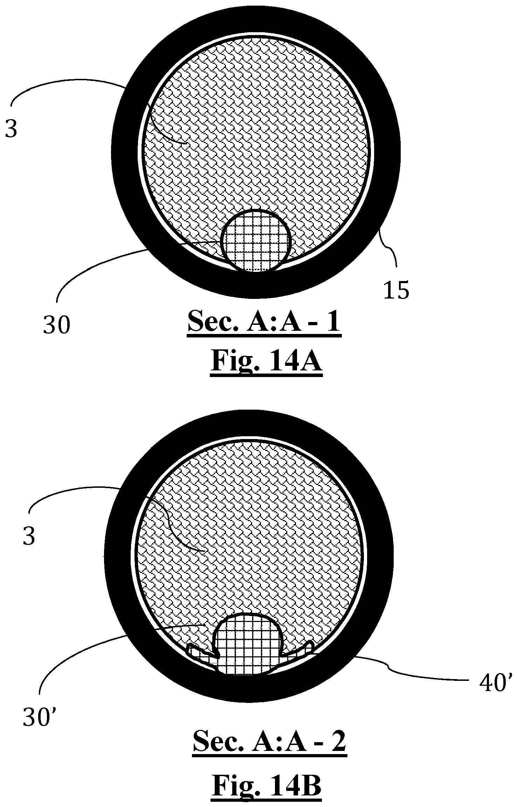

Fig. 14A is a view of a guidewire 30 from FIG. 13 along section A-A. In particular, in this view it can be seen that guidewire 30 is rounded and has been advanced across the occlusion 3. In contrast, guidewire 30' of FIG. 14B depicts a rounded guidewire with a planar, flattened distal tip 40' that is capable of being orientated in only two configurations, as explained more particularly below.

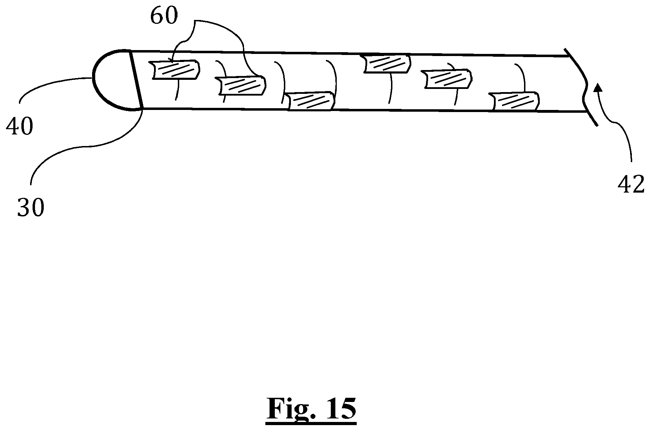

FIG. 15 is an illustration of an example guidewire 30 with sensors 60 for detecting properties of clot 30, as previously shown in FIG. 14A. In particular, FIG. 15 depicts six (6) sensors that are substantially rectangular and circumferentially spaced about an outer surface of guidewire 30. The sensors 60 are shown extending proximally from distal tip 40 towards the proximal end of guidewire 30 (see, e.g., FIG. 1). However, the solution of FIG. 15 is not so limited and any number of sensors, whether fewer or more, are contemplated for use as needed or required. Sensor 60 may also be configured with any shape as needed or required. It is understood that guidewire 30 is a conventional guidewire and that sensors 60 could be any of those contemplated within this disclosure, including NIR, Raman spectroscopy, impedance sensors, or fiberoptic strands or bundles operable to transmit and collect certain ranges of the electromagnetic spectrum.

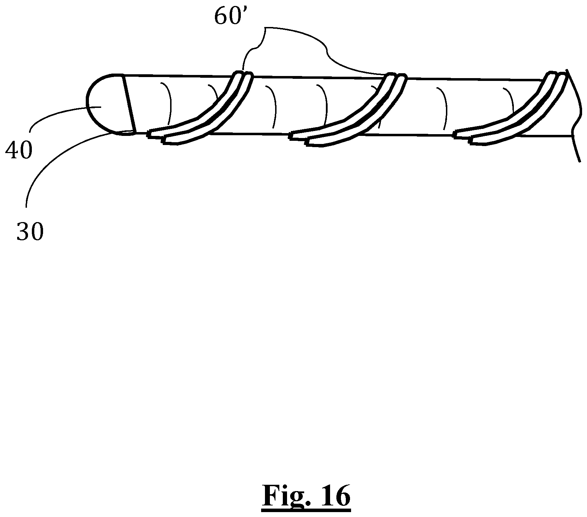

FIG. 16 is an illustration of an example guidewire 30 with sensors 60' for detecting properties of clot 30, as previously shown in FIG. 14A. In particular, FIG. 16 depicts sensors 60' that are circumferentially wrapped around and/or spaced about an outer surface of guidewire 30. The sensors 60', similar to sensors 60, are shown extending proximally from distal tip 40 towards the proximal end (not depicted) of guidewire 30. However, the solution of FIG. 16 is not so limited and any number of sensors, whether fewer or more, are contemplated for use as needed or required. Sensor 60 may also be configured with any shape as needed or required.

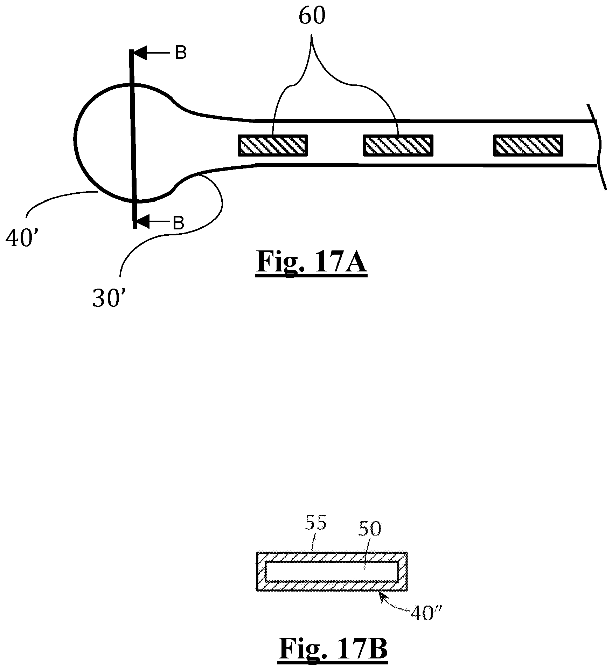

Fig. 17A is an exemplary illustration of an atraumatic clot-circumventing configured distal tip 40' of a guidewire 30'. Tip 40' can be a flattened portion of the core wire cut or stamped into a paddle shape, as described more particularly in U.S. Pat. App. 16/003,527, which is incorporated by reference herein in its entirety as if set forth verbatim. As can be seen, the paddle shaped distal tip 40' is in a relaxed or non-compressed state (not subject to application of an external mechanical force). Rather than pre-forming or pre-shaping the guidewire 30' to a desired geometric shape, the distal tip 40'can have a circular lateral cross-section and/or may alternatively be flattened out and then cut or stamped into a desired geometric shape to form the atraumatic clot-circumventing configured distal end or tip (e.g., as the depicted "planar paddle shape" 40').

Because the guidewire 30 discussed previously in FIGs. 15-16 is round, there is no easy way to control the orientation of the guidewire 30 as to the clot 3 and any sensors 60. To resolve this problem, planar guidewire 30' is basically configured to be oriented only in the depicted flat or planar position of FIG. 17A or a collapsed condition prior to deployment. This is particularly advantageous with guidewire 25' since sensors 60 would be caused to face the clot 60 during use, rather than rotating or moving or otherwise moving in a manner that causes sensors 60 to misread or inaccurately sense properties of clot 60. For example, the sensors 60 of previous FIGs. 15-16 may at times face the vessel wall 15 whereas other times may be half way between the walls 15 and clot 3. To resolve this, rather than developing a complex algorithm to sort out and process the resultant signals, which may not be easy to do and which result in reduced sensing accuracy, the herein disclosed planar guidewire 30', including planar tip 40', can be operable to move between one of two orientations as it crosses the clot as shown in FIG. 14B.

Turning back to FIG. 17A, sensors 60 can be advantageously positioned on both sides of the guidewire 30' and/or aligned with tip 40'. In turn, the resultant clot sensing signals detected through sensors 60 will be much easier to distinguish and process since they will be oriented as either fully facing the clot 3 or fully facing the vessel wall 15.

In this respect, distal tip 40' can be flattened to form a thin support structure or substrate 50, typically having a thickness ranging from approximately 0.0005" to approximately 0.010", preferably the thickness ranges from approximately 0.001" to approximately 0.005". Tip 40'may alternatively be achieved by assembling a pre-formed planar paddle shaped distal end or tip, having the same specified thickness range, on an end of a separate core wire, such as by welding, soldering or bonding. Two separate components assembled or joined together is particularly well suited to applications in which the pre-formed planar paddle and core wire on which the paddle is to be assembled are made of different materials.

As illustrated in the cross-sectional view in Fig. 17B taken along section B-B of Fig. 17A. An outer covering or layer 55 may be applied to the external surface of the supporting structure 50. Outer covering or layer 55 may comprise one or more coverings, coating layers, or films. For instance, the outer covering or layer 55 may comprise a polymer jacket applied directly to the outer surface of the support structure or substrate 50 with or without a lubricious coating, e.g., a hydrophilic material, applied directly to the outer surface of the polymer jacket. The outer jacket may also contain radiopaque fillers to enhance fluoroscopic visibility of the tip.

The guidewire 30' may be retracted or drawn back through the distal end 20 of the microcatheter 10. In this respect, the distal tip 40' of guidewire 30' can move between collapsed to expanded configuration in a manner akin to that of rolling one's tongue (e.g., in the collapsed configuration, lateral edges of distal end 40' can be in a curled or curved or wrapped state and then can curl or roll towards one another during deployment to the expanded, paddle configuration). Upon exiting from the lumen 25 of the microcatheter 10, the paddle shaped distal end or tip 40" of the guidewire is automatically restored to its former planar relaxed or non-compressed state (not subject to application of an external mechanical force) (as seen in FIG. 17A).

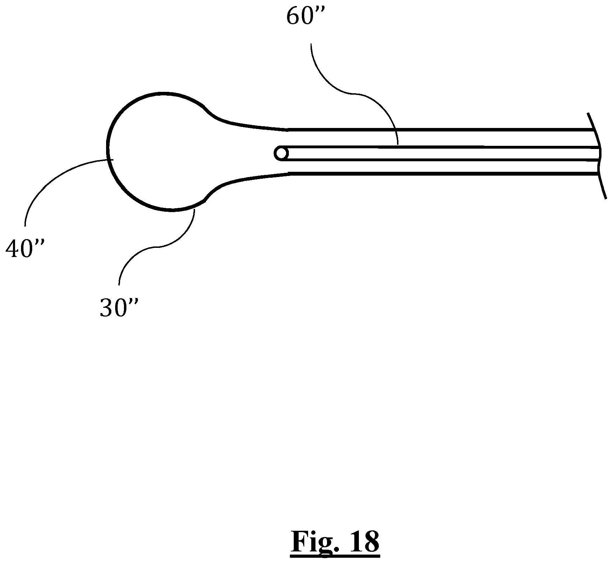

Fig. 18 is an exemplary illustration of an atraumatic clot-circumventing configured distal tip 40" of a guidewire 30", similar to tip 40' of guidewire 30' except for that the guidewire 30" of Fig. 18 includes fiber sensor 60" that can be aligned with the planar, flattened surface of guidewire 30".

In other embodiments, a temperature measurement device is disclosed for sensing temperature readings at different locations in the vasculature, including along and inside a clot. The device can be configured to identify, among other information, clot type or composition of the clot generally or of internal and external regions of the clot. In some examples, the device can be configured to determine clot heterogeneity along the length of the clot. In some examples, the device can be configured to characterize the clot *in vivo,* for example, to a determine the origin of the clot (e.g., based on temperature readings of the clot it can be determined that the clot is from an atrial appendage or ruptured atherosclerotic plaque).

In some examples, the temperature measurement device can be part of a system for analyzing temperature differentials that in turn can utilize the temperature readings with respect to the clot and surrounding areas to provide information related to the clot to improve treatment. In some embodiments, device can include one or more sensors for sensing temperature heterogeneity in and around a clot. In turn, the system is configured to correlate the sensed temperature from device with differences in properties of the clot, including physical and/or chemical properties, clotting mechanisms, time of clot formation, and the like. By detecting temperature heterogeneity of the clot, as well as other intravascular temperatures (e.g., at vessel wall, in regions of restricted and unrestricted flow, etc.), the system can further characterize the clot for improving diagnosis and treatment for the patient. Temperature readings by device can also be used by system to correlate and identify vulnerable plaque in and surrounding the clot to similarly characterize the clot. In some embodiments, device may also be configured to measure the pH of the clot and its surrounding areas for similar analysis of the clot.

The temperature measurement device can be in the form of a guidewire or a catheter configured to precisely measure the temperature at multiple points within a clot, along the length of the clot, at positions of the vessel wall at or adjacent the clot, proximal to the face of the clot, and distal to a clot. In some examples, the distal end of the device can be used to take these temperature measurements compared with a reference temperature at a location proximal to the clot and in a region of unrestricted blood flow. The location proximal could be at the mid-length point of the device or it could be at a point that is proximal of a bifurcation (e.g., carotid bifurcation where the clot is in one of the vessels coming off the bifurcation). It is contemplated that the temperature within the clot would be higher than the temperature in a region which has blood flow. In this respect, a difference in temperature between normal flowing blood and the blood in the region surrounding the clot can be used to characterize the clot. The device could also be used to measure the temperature of the vessel wall in the region of the clot.

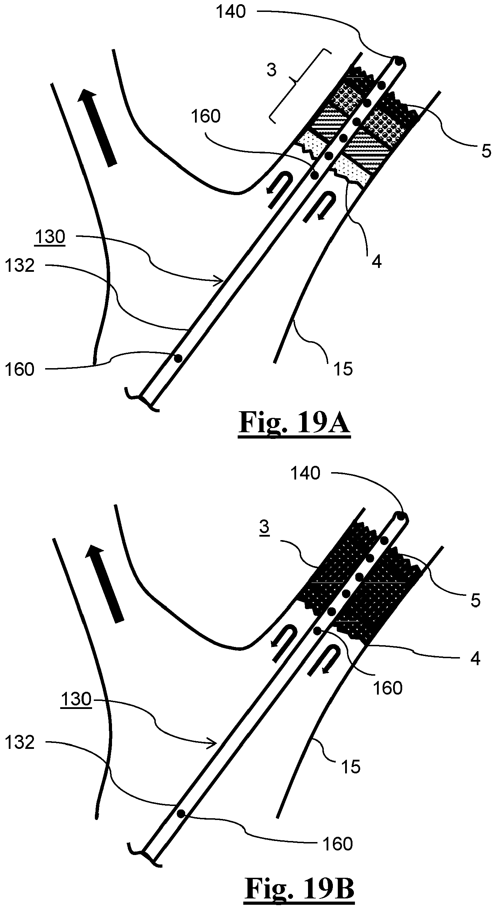

The temperature measurement device could include one or more sensors embedded in a guidewire or catheter. In some examples, the device could have a single sensor at the distal tip and the temperature could be measured at different points by moving the device tip to different locations. By measuring a temperature differential between points, a relatively small region of the clot can be characterized using a minute thermocouple junction. Examples of temperature measurement devices are shown in Figs. 19A-21A.

In particular, in Figs. 19A-B, one example of a temperature measurement device is depicted with guidewire 130. Guidewire 130 can be a conventional guidewire with an atraumatic tip and be extended into the vasculature distally and through or about clot 3. Clot 3 in Fig. 19A is shown having an example gradient with discrete sections merely as an illustration of typical clots that vary their physical properties throughout, including from proximal face 4 and distal face 5 of clot 3. Clot 3 in Fig. 19B is shown in having uniform color, though this does not necessarily mean that clot 3 is homogenous or has any more or less of a particular property.

Guidewire 130 can be substantially elongate yet flexible to be able to be translated through the vasculature to the site of the clot. The arrows shown are intended to depict example blood flow in vessel 15. Guidewire 130 can include a single temperature sensor 160 or a plurality of sensors 160 as shown. In Fig. 19A, guidewire 130 includes one sensor 160 in the unrestricted flow region 132 of vessel 15, though more than a single sensor 160 could be used as needed or required to provide desired accuracy of the region 132. On or about the distal end 140 of guidewire 130, one or more additional sensors 160 can also be positioned. Sensors 160 can be selectively to correspond to locations of the clot 3. As shown, end 140 and corresponding sensors 160 can be translated distally until end 140 is distal of distal face 5 and to take temperature measurements both within the clot as well as proximal and distal thereof.

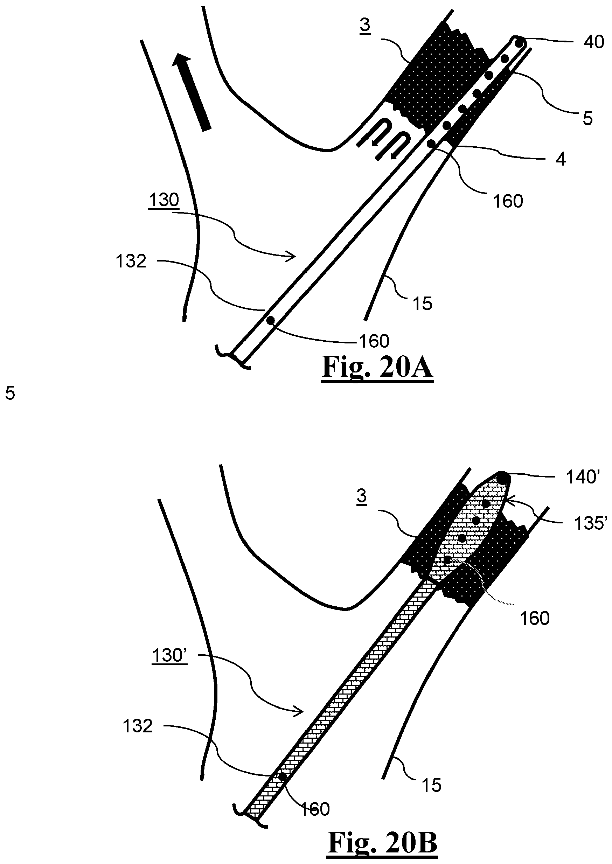

In Fig. 20A, distal end 140 of guidewire 130 has been translated laterally to one side of the clot so that one or more of its corresponding sensors 160 are positioned adjacent the vessel wall. In this regard, guidewire 130 can detect temperature of the vessel wall for use in further analyzing properties of clot 3 and the surrounding vasculature.

Turning to Fig. 20B, a modified guidewire 130' is depicted, similar to planar guidewire previously discussed in Figs. 17A-19. In this respect, guidewire 130' can include one or more sensors 160 positioned proximal of the clot 3 and about the distal end 140'. Distal end 140' can include a curved or expanded perimeter capable of being in an expanded configuration. Guidewire 130' can be in an expanded configuration as shown as a result of being deployed from a catheter (not depicted), from being actuated by an end user from the proximal end of guidewire 130', etc. In turn, while only six (6) sensors 160 are depicted in Fig. 20B, the distal end 140' with its expanded distal end 140' can include multiple temperature sensors 160 so more simultaneous temperature measurements can be taken throughout the clot 3 for more precise clot characterization.

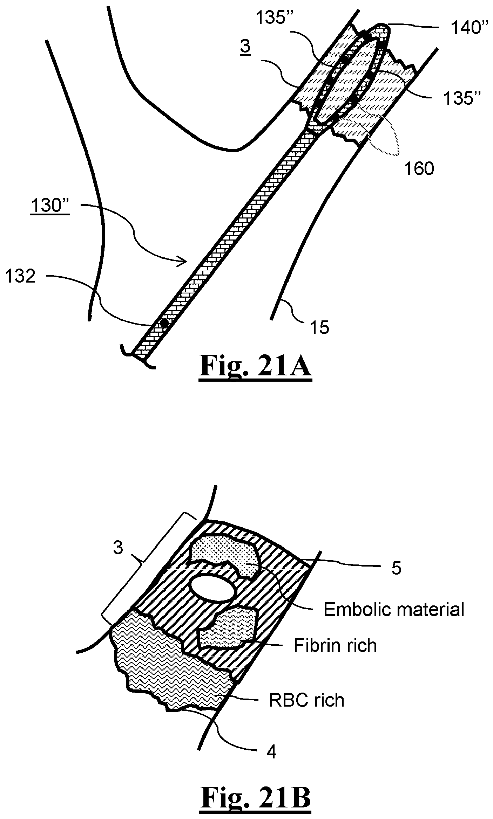

Turning to Fig. 21A, another modified guidewire 130" is depicted, similar to guidewire 130'. In this respect, guidewire 130" can include one or more sensors 160 positioned proximal of the clot 3 and about the distal end 140". Distal end 140" in this embodiment can include multiple loops 135" that define a curved or expanded perimeter with a gap or space formed between the respective loops 135" in the expanded configuration. Each loop 135" of distal end 140" can include multiple temperature sensors 160 so more simultaneous temperature measurements can be taken throughout the clot 3 for more precise clot characterization, including at multiple locations within the clot 3.

Fig. 21B is a close-up view of one example of a clot 3 as it could be analyzed by guidewire 130 / 130'. For example, in clot 3 of Fig. 21B it can be seen how heterogenous clot 3 can be in terms of its material composition. In the depicted example, clot 3 includes multiple regions with differing properties, such as a proximal RBC rich region, a central fibrin rich region, and distally located embolic material within the clot 3. The solutions of this disclosure are particularly advantageous since they can be used to detect *in vivo* the different material properties of clot 3, including any regions internal therein, to then analyze and determine the optimal treatment device(s) and corresponding treatment protocol for the patient.

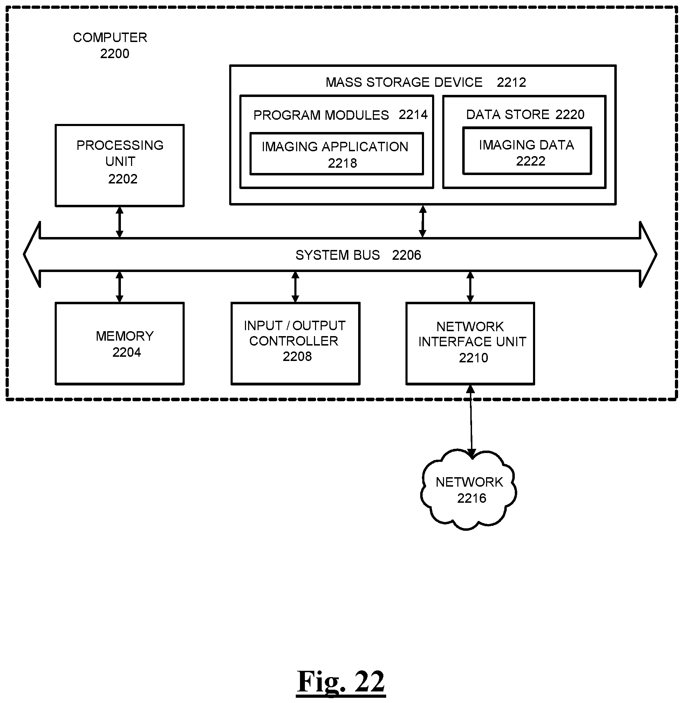

FIG. 22 is a computer architecture diagram showing a general computing system capable of implementing aspects of the present disclosure in accordance with one or more embodiments described herein, including clot analysis and/or clot classification and/or individualizing a treatment protocol for a patient. A computer 2200 may be configured to perform one or more functions associated with embodiments of this disclosure. For example, the computer 2200 may be configured to perform operations in order to render the imaging of associated with the herein disclosed embodiments related to clot analysis and/or classification and/or individualizing treatment protocol for a respective patient. It should be appreciated that the computer 2200 may be implemented within a single computing device or a computing system formed with multiple connected computing devices. The computer 2200 may be configured to perform various distributed computing tasks, in which processing and/or storage resources may be distributed among the multiple devices. The data acquisition and display computer 2250 and/or operator console 2210 of the system shown in FIG. 22 may include one or more systems and components of the computer 2200.

As shown, the computer 2200 includes a processing unit 2202 ("CPU"), a system memory 2204, and a system bus 2206 that couples the memory 2204 to the CPU 2202. The computer 2200 further includes a mass storage device 2212 for storing program modules 2214. The program modules 2214 may be operable to analyze and/or classify one or more clots in vivo and/or in vitro, as well as individualize treatment of a patient, discussed herein. For example, to cause the computer 2200 to a clot of a patient as described in any of the figures of this disclosure. The program modules 2214 may include an imaging application 2218 for performing data acquisition and/or processing functions as described herein, for example to acquire and/or process image data corresponding to magnetic resonance imaging of an area of interest. The computer 2200 can include a data store 2220 for storing data that may include imaging-related data 2222 such as acquired data from the implementation of magnetic resonance imaging in accordance with various embodiments of the present disclosure.

The mass storage device 2212 is connected to the CPU 2202 through a mass storage controller (not shown) connected to the bus 2206. The mass storage device 2212 and its associated computer-storage media provide non-volatile storage for the computer 2200. Although the description of computer-storage media contained herein refers to a mass storage device, such as a hard disk or CD-ROM drive, it should be appreciated by those skilled in the art that computer-storage media can be any available computer storage media that can be accessed by the computer 2200.

By way of example and not limitation, computer storage media (also referred to herein as "computer-readable storage medium" or "computer-readable storage media") may include volatile and non-volatile, removable and non-removable media implemented in any method or technology for storage of information such as computer-storage instructions, data structures, program modules, or other data. For example, computer storage media includes, but is not limited to, RAM, ROM, EPROM, EEPROM, flash memory or other solid state memory technology, CD-ROM, digital versatile disks ("DVD"), HD-DVD, BLU-RAY, or other optical storage, magnetic cassettes, magnetic tape, magnetic disk storage or other magnetic storage devices, or any other medium which can be used to store the desired information and which can be accessed by the computer 2200. "Computer storage media", "computer-readable storage medium" or "computer-readable storage media" as described herein do not include transitory signals.

According to various embodiments, the computer 2200 may operate in a networked environment using connections to other local or remote computers through a network 2216 via a network interface unit 2210 connected to the bus 2206. The network interface unit 2210 may facilitate connection of the computing device inputs and outputs to one or more suitable networks and/or connections such as a local area network (LAN), a wide area network (WAN), the Internet, a cellular network, a radio frequency (RF) network, a Bluetooth-enabled network, a Wi-Fi enabled network, a satellite-based network, or other wired and/or wireless networks for communication with external devices and/or systems.

The computer 2200 may also include an input/output controller 2208 for receiving and processing input from any of a number of input devices. Input devices may include one or more of keyboards, mice, stylus, touchscreens, microphones, audio capturing devices, and image/video capturing devices. An end user may utilize the input devices to interact with a user interface, for example a graphical user interface, for managing various functions performed by the computer 2200. The bus 2206 may enable the processing unit 2202 to read code and/or data to/from the mass storage device 2212 or other computer-storage media.

The computer-storage media may represent apparatus in the form of storage elements that are implemented using any suitable technology, including but not limited to semiconductors, magnetic materials, optics, or the like. The computer-storage media may represent memory components, whether characterized as RAM, ROM, flash, or other types of technology. The computer storage media may also represent secondary storage, whether implemented as hard drives or otherwise. Hard drive implementations may be characterized as solid state or may include rotating media storing magnetically-encoded information. The program modules 2214, which include the imaging application 2218, may include instructions that, when loaded into the processing unit 2202 and executed, cause the computer 2200 to provide functions associated with one or more embodiments illustrated in the figures of this disclosure. The program modules 2214 may also provide various tools or techniques by which the computer 2200 may participate within the overall systems or operating environments using the components, flows, and data structures discussed throughout this description.

In general, the program modules 2214 may, when loaded into the processing unit 2202 and executed, transform the processing unit 2202 and the overall computer 2200 from a general-purpose computing system into a special-purpose computing system. The processing unit 2202 may be constructed from any number of transistors or other discrete circuit elements, which may individually or collectively assume any number of states. More specifically, the processing unit 2202 may operate as a finite-state machine, in response to executable instructions contained within the program modules 2214. These computer-executable instructions may transform the processing unit 2202 by specifying how the processing unit 2202 transitions between states, thereby transforming the transistors or other discrete hardware elements constituting the processing unit 2202.

Encoding the program modules 2214 may also transform the physical structure of the computer-storage media. The specific transformation of physical structure may depend on various factors, in different implementations of this description. Examples of such factors may include but are not limited to the technology used to implement the computer-storage media, whether the computer storage media are characterized as primary or secondary storage, and the like. For example, if the computer storage media are implemented as semiconductor-based memory, the program modules 2214 may transform the physical state of the semiconductor memory, when the software is encoded therein. For example, the program modules 2214 may transform the state of transistors, capacitors, or other discrete circuit elements constituting the semiconductor memory.

As another example, the computer storage media may be implemented using magnetic or optical technology. In such implementations, the program modules 2214 may transform the physical state of magnetic or optical media, when the software is encoded therein. These transformations may include altering the magnetic characteristics of particular locations within given magnetic media. These transformations may also include altering the physical features or characteristics of particular locations within given optical media, to change the optical characteristics of those locations. Other transformations of physical media are possible without departing from the scope of the present description, with the foregoing examples provided only to facilitate this discussion.

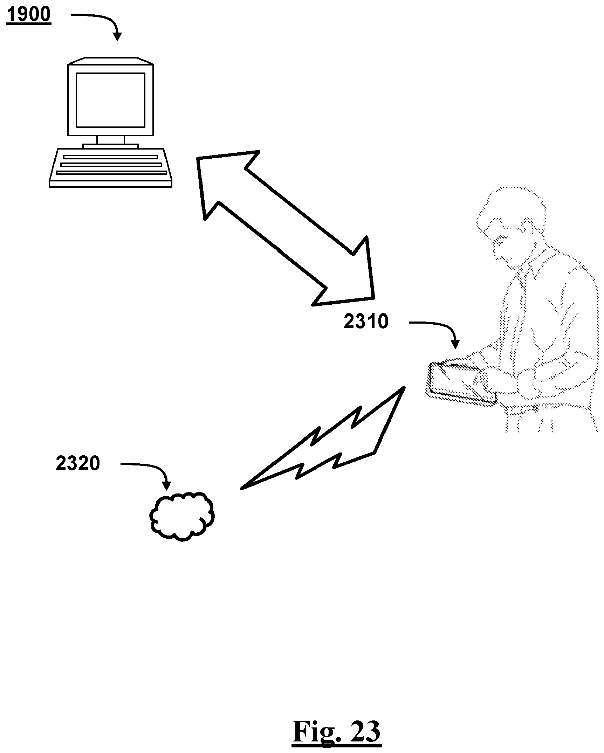

FIG. 23 depicts a schematic overview of an example embodiment of a mobile device 2310 analyzing a clot 2320 or fragments of a clot 2320 in vitro recently removed from the patient. The mobile device 2310 may be any electronic device configured to capture images, such as a mobile phone, media player, portable gaming device, tablet computer, or the like. It is noted that the present disclosure is not limited to any single type of mobile device. Device 2310 can be wirelessly connected and controlled by an external computing device and/or system, such as system 2200, whereby such external system can be operable to execute instructions related to analysis and/or classification and/or individualization of a treatment protocol for the clot, according to any of the previously disclosed embodiments of this disclosure. Alternatively, device 2310 can carry out the analysis and/or classification and/or individualization of a treatment protocol for a patient and their respective clot, locally.

Device 2310 may operatively communicate with the external computing device through an application resident on device 2310. Device 2310 may include an optical system, such as an onboard camera, configured to capture images or video of clot 2320 or fragments of a clot 2320 in order to analyze and/or classify the same.

Exemplary architecture of device 2310 can include a central processing unit, where computer instructions are processed; a display interface that acts as a communication interface and provides functions for rendering video, graphics, images, and texts on the display and a keyboard interface that provides a communication interface to a keyboard; and a pointing device interface that provides a communication interface to device 2310 and/or any external computing devices coupled thereto. Example embodiments of the architecture may include an antenna interface that provides a communication interface to an antenna. Example embodiments may include a network connection interface that may provide a communication interface to an external device or network.

In certain embodiments, a camera interface may be provided that acts as a communication interface and provides functions for capturing digital images from the onboard camera and capabilities of visualizing certain aspects of the clot, including levels of fibrin, red blood cells, white blot cells, serum, and other physical properties. According to example embodiments, a random access memory (RAM) may be provided, where computer instructions and data may be stored in a volatile memory device for processing by the CPU. The architecture may include a read-only memory (ROM) where invariant low-level system code or data for basic system functions such as basic input and output (I/O), startup, or reception of keystrokes from a keyboard are stored in a non-volatile memory device. According to an example embodiment, the architecture may include a storage medium or other suitable type of memory (e.g. such as RAM, ROM, programmable read-only memory (PROM), erasable programmable read-only memory (EPROM), electrically erasable programmable read-only memory (EEPROM), magnetic disks, optical disks, floppy disks, hard disks, removable cartridges, flash drives), where the files include an operating system, application programs (including, for example, a web browser application, a widget or gadget engine, and or other applications, as necessary) and data files are stored. According to an example embodiment, the architecture may include a power source that provides an appropriate alternating current (AC) or direct current (DC) to power components. According to an example embodiment, the architecture may include a telephony subsystem that allows the device to transmit and receive sound over a telephone network. The constituent devices and the CPU may communicate with each other over a bus.

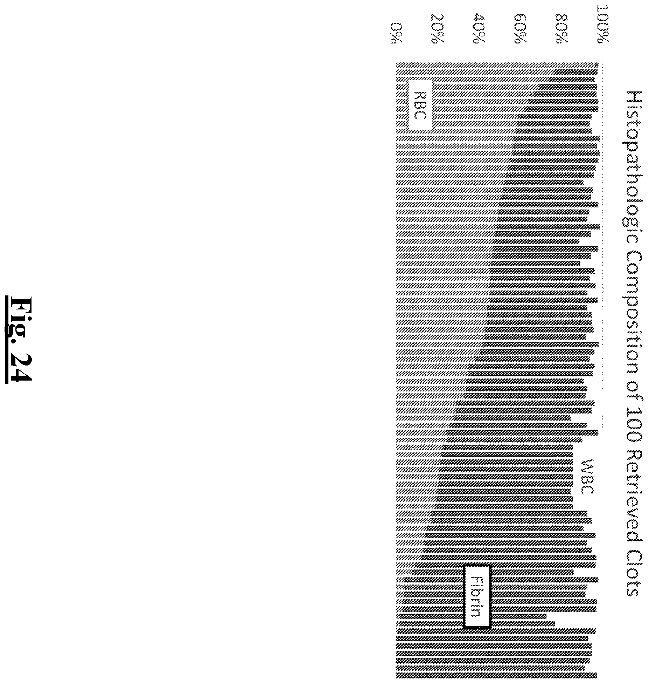

FIG. 24 depicts an overview of histopathologic composition of 100 clots analyzed in this disclosure. The clot analysis for each clot has been sorted from highest to lowest red blood cell (RBC) count and lowest to highest fibrin count. FIG. 24 shows significant variance of even just three clot physical properties across 100 clots tested in vitro that requires different treatment protocols depending on such identified physical properties.

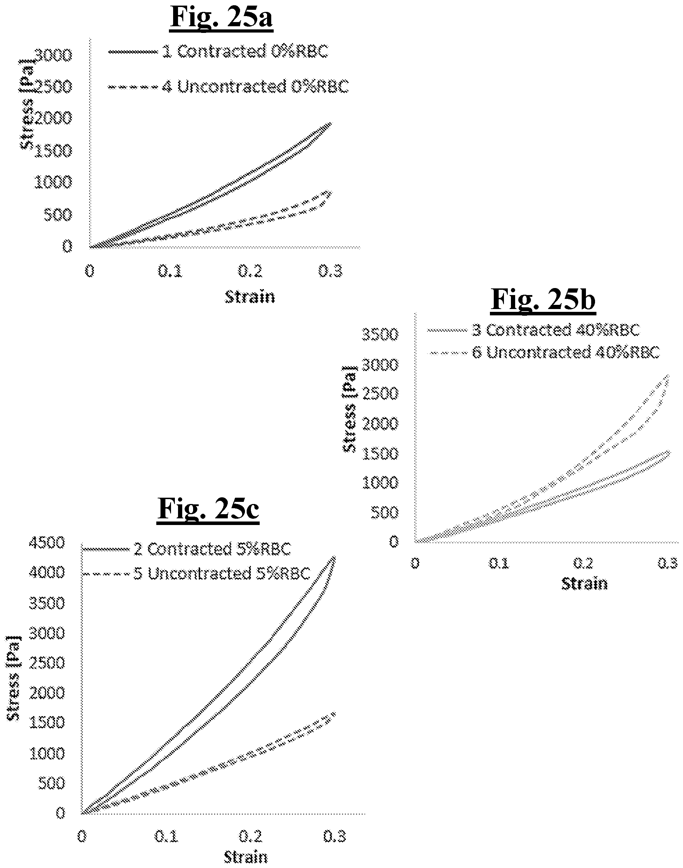

FIGs. 25a-c depict graphs that compare the strain rate for three separate clots classified by red blood cell count. Specifically, FIG. 25a depicts a graph showing the strain rate for a clot in contracted and uncontracted states identified as having 0% red blood cell count, FIG. 25b depicts a graph showing the strain rate for a clot in contracted and uncontracted states identified as having 40% red blood cell count, and FIG. 25c depicts a graph showing the strain rate for a clot in contracted and uncontracted states identified as having 5% red blood cell count. As can be seen, as red blood cell count varies in the clots that were analyzed, platelet activated contraction of the clot changes its corresponding stiffness and in turn the treatment necessary to restore perfusion to a vessel occluded by said clot. The solution of this disclosure is configured to treat patients across the depicted red blood cell accounts, classify, analyze, and determine the treatment protocol for the individual with the occluded vessel.

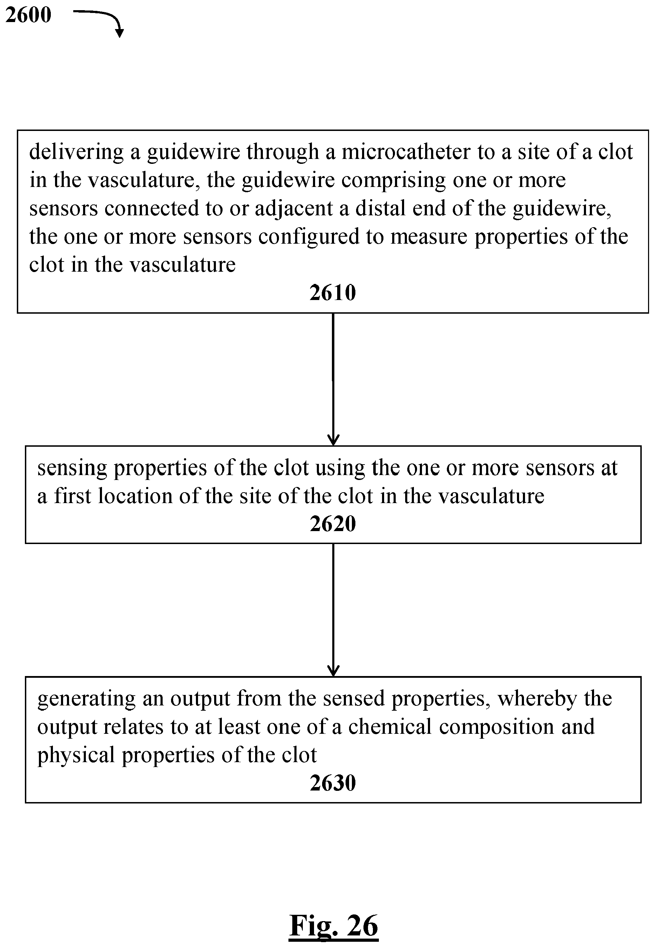

FIG. 26 shows an example method 2600 for managing one or more acute ischemic events. The method can include step 2610 delivering a guidewire through a microcatheter to a site of a clot in the vasculature, the guidewire comprising one or more sensors connected to or adjacent a distal end of the guidewire, the one or more sensors configured to measure properties of the clot in the vasculature. The method can also include 2620 sensing properties of the clot using the one or more sensors at a first location of the site of the clot in the vasculature. The method can also include step 2630 generating an output from the sensed properties, whereby the output relates to at least one of a chemical composition and physical properties of the clot.

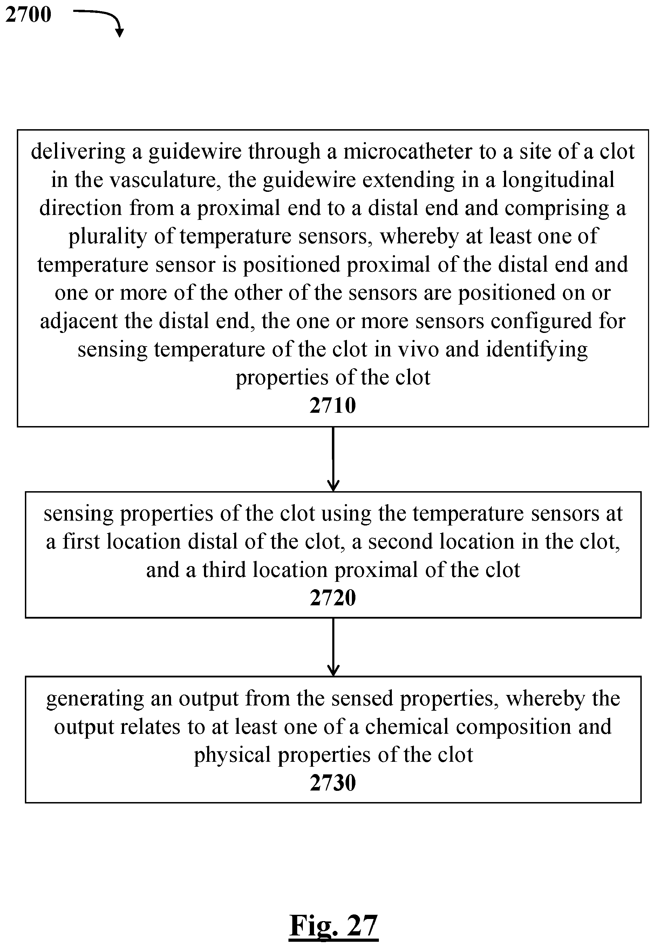

FIG. 27 shows an example method 2700 for managing one or more acute ischemic events. The method can include step 2710 delivering a guidewire through a microcatheter to a site of a clot in the vasculature, the guidewire extending in a longitudinal direction from a proximal end to a distal end and comprising a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot. Step 2720 can include sensing properties of the clot using the temperature sensors at a first location distal of the clot, a second location in the clot, and third location proximal of the clot. Step 2730 can include generating an output from the sensed properties, whereby the output relates to at least one of a chemical composition and physical properties of the clot.

It must also be noted that, as used in the specification and the appended claims, the singular forms "a," "an" and "the" include plural referents unless the context clearly dictates otherwise. Ranges may be expressed herein as from "about" or "approximately" one particular value and/or to "about" or "approximately" another particular value. When such a range is expressed, other exemplary embodiments include from the one particular value and/or to the other particular value.

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the present disclosure. Similarly, it is also to be understood that the mention of one or more components in a device or system does not preclude the presence of additional components or intervening components between those components expressly identified.

Some references, which may include various patents, patent applications, and publications, are cited in a reference list and discussed in the disclosure provided herein. The citation and/or discussion of such references is provided merely to clarify the description of the present disclosure and is not an admission that any such reference is "prior art" to any aspects of the present disclosure described herein. In terms of notation, "[n]" corresponds to the n^{th} reference in the list. All references cited and discussed in this specification are incorporated herein by reference in their entireties and to the same extent as if each reference was individually incorporated by reference.

The descriptions contained herein are examples illustrating the solution and are not intended to limit the scope. As described herein, the solution contemplates many variations and modifications of a system, device, and/or method that can be used to analyze one or more clots and individualize treatment based on the analysis. Variations can include but are not limited to alternative geometries of elements and components described herein, utilizing any of numerous materials for each component or element (e.g. radiopaque materials, memory shape metals, etc.), utilizing additional components, utilizing additional components to perform functions described herein, or utilizing additional components to perform functions not described herein, for example. These modifications would be apparent to those having ordinary skill in the art to which this invention relates and are intended to be within the scope of the claims which follow.

The specific configurations, choice of materials and the size and shape of various elements can be varied according to particular design specifications or constraints requiring a system or method constructed according to the principles of the disclosed technology. Such changes are intended to be embraced within the scope of the disclosed technology. The presently disclosed embodiments, therefore, are considered in all respects to be illustrative and not restrictive. It will therefore be apparent from the foregoing that while particular forms of the disclosure have been illustrated and described, various modifications can be made without departing from the spirit and scope of the disclosure and all changes that come within the meaning and range of equivalents thereof are intended to be embraced therein.

### ASPECTS OF THE INVENTION

1. A method for managing one or more acute ischemic events, the method comprising:
   delivering a guidewire through a microcatheter to a site of a clot in the vasculature, the guidewire comprising one or more sensors connected to or adjacent a distal portion of the guidewire, the one or more sensors configured to measure properties of the clot in the vasculature;
   sensing properties of the clot using the one or more sensors at a first location of the site of the clot in the vasculature; and
   generating an output from the sensed properties, whereby the output relates to at least one of a chemical composition and physical properties of the clot.
2. The method according to aspect 1, further comprising: selecting one or more devices and/or procedural steps to treat the clot based on the output.
3. The method according to aspect 1, wherein the distal portion of the guidewire is an atraumatic clot-circumventing configured distal end, and wherein the one or more sensors are circumferentially disposed or wrapped about an outer surface of the guidewire.
4. The method according to aspect 1, wherein the distal portion of the guidewire is a flattened distal portion having a planar geometric shape and thickness less than an outer diameter of a remaining non-flattened portion.
5. The method according to aspect 1, further comprising:
   positioning at least one of the one or more sensors on a flattened distal portion for sensing properties of the clot; and
   positioning the other of the one or more sensors on the remaining non-flattened portion for sensing properties of the vessel wall.
6. The method according to aspect 1, further comprising:
   classifying the clot based on the sensed properties and generating a classification.
7. The method according to aspect 1, wherein the one or more sensors are near infrared (NIR) sensors disposed on an outer surface of the guidewire.
8. The method according to aspect 1, wherein the one or more sensors are impedance sensors disposed on an outer surface of the guidewire.
9. The method according to aspect 1, wherein the one or more sensors are impedance sensors disposed on an outer surface of the guidewire.
10. The method according to aspect 1, wherein the one or more sensors are Raman spectroscopy sensors disposed on an outer surface of the guidewire and operable to transmit and collect certain ranges of the electromagnetic spectrum.
11. The method according to aspect 1, wherein the one or more sensors are one or more fiberoptic strands or bundles disposed on an outer surface of the guidewire and operable to transmit and collect certain ranges of the electromagnetic spectrum.
12. The method according to aspect 1, further comprising:
   ascertaining one or more physical properties of the clot by measuring different spectral features of the clot based on the output.
13. The method according to aspect 1, further comprising: interpreting information contained in the output to determine in real-time at least one of levels of red blood cell content, white blood cell content, platelet content, levels of fibrin, a clot size, a clot shape, a clot density, and a clot location in the vasculature.
14. The method according to aspect 1, further comprising:
   sensing properties of the clot using the one or more sensors at a second location distal or proximal of the clot and the first location; and
   generating an output from the second location, whereby the output of the second location relates to at least one of a chemical composition and physical properties of the clot.
15. The method according to aspect 1, further comprising: classifying the clot based on the output and one or more of a clinical exam, a blood test, a computerized tomography (CT) scan, a magnetic resonance imaging (MRI) scan, an ultrasound scan, a cerebral angiogram, and an echocardiogram.
16. The method according to aspect 1, further comprising: determining criteria of the clot by
   performing at least one of a CT scan and an MRI scan; and
   analyzing information from at least one of the CT scan and the MRI scan to determine physical and/or chemical composition of the clot, including levels of red blood cell content, white blood cell content, levels of fibrin, a clot size, a clot shape, and/or a clot location in the vasculature.
17. The method according to aspect 1, further comprising: determining criteria of the clot by
   determining one or more quantitative indications selected from one or more of white blood cell levels, red blood cell levels, serum levels, fibrin levels, clot size, clot location, clot strength, clot elasticity, rate of clot formation or rate of clot lysis.
18. A method for managing one or more acute ischemic events, the method comprising:
   delivering a guidewire through a microcatheter to a site of a clot in the vasculature, the guidewire extending in a longitudinal direction from a proximal end to a distal end and comprising a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot;
   sensing properties of the clot using at least one of the temperature sensors at a first location distal of the clot, a second location in the clot, and a third location proximal of the clot; and
   generating an output from the sensed properties, whereby the output relates to at least one of a chemical composition and physical properties of the clot.
19. The method according to aspect 18, wherein the step of sensing properties with the temperature sensors is done simultaneously at each location.
20. The method according to aspect 18, further comprising: selectively positioning the sensors at predetermined locations along the distal end to measure regions of the clot.
21. The method according to aspect 18, further comprising:
   separating each sensor a predetermined distance at locations along the distal end to measure regions of the clot.
22. The method according to aspect 18, wherein the distal end of the guidewire comprises an expanded perimeter with an atraumatic clot-circumventing configured distal end in a delivery configuration, and wherein the one or more sensors are disposed about the expanded perimeter.
23. The method according to aspect 18, further comprising: classifying the clot based on the sensed properties and generating a classification.
24. The method according to aspect 18, further comprising: interpreting information contained in the output to determine in real-time at least one of levels of red blood cell content, white blood cell content, levels of fibrin, a clot size, a clot shape, and a clot location in the vasculature.
25. The method according to aspect 18, further comprising: classifying the clot based on the output and one or more of a clinical exam, a blood test, a computerized tomography (CT) scan, a magnetic resonance imaging (MRI) scan, a carotid ultrasound, a cerebral angiogram, and an echocardiogram.
26. The method according to aspect 18, further comprising:
   determining criteria of the clot by
   performing at least one of a CT scan and an MRI scan; and
   analyzing information from at least one of the CT scan and the MRI scan to determine physical and/or chemical composition of the clot, including levels of red blood cell content, white blood cell content, levels of fibrin, a clot size, a clot shape, and/or a clot location in the vasculature.
27. A method for managing one or more acute ischemic events, the method comprising:
   delivering a guidewire through a microcatheter to a site of a clot in the vasculature, the guidewire extending in a longitudinal direction from a proximal end to a distal end and comprising a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot;
   sensing properties of the clot using the temperature sensors at a plurality of locations in the clot; and
   generating an output from the sensed properties, whereby the output relates to at least one of a chemical composition and physical properties of the clot.
28. The method according to aspect 27, further comprising:
   sensing properties of the clot at a location distal and/or proximal of the clot.

## Claims

1. An endovascular medical system for use with a clot located in a target vessel for managing one or more acute ischemic events, the system comprising:
a guidewire extending in a longitudinal direction from a proximal end to a distal end, the distal end being configured to control its orientation relative to the clot and the target vessel;,
wherein the guidewire comprises one or more sensors disposed on or adjacent the distal end, the one or more sensors configured for sensing properties of the clot *in vivo* and treating the clot based on the sensed properties.

2. The system according to Claim 1, wherein the distal end of the guidewire is configured to prevent injury to an inner wall of the target vessel.

3. The system according to Claim 1, wherein the one or more sensors are impedance sensors disposed on an outer surface of the guidewire, or one or more fiberoptic strands or bundles disposed on an outer surface of the guidewire and operable to transmit and collect certain ranges of the electromagnetic spectrum.

4. The system according to Claim 1, wherein the distal end of the guidewire is an atraumatic clot-circumventing configured distal end, and wherein the one or more sensors are circumferentially disposed about an outer surface of the guidewire, or a flattened distal portion having a planar geometric shape and thickness less than an outer diameter of a remaining non-flattened portion.

5. The system according to Claim 4 when the distal end of the guidewire is a flattened distal portion, wherein
at least one of the one or more sensors is disposed on the flattened distal portion for sensing properties of the clot; and
the other of the one or more sensors is disposed on the remaining non-flattened portion for sensing properties of the vessel wall.

6. The system according to Claim 4 when the distal end of the guidewire is a flattened distal portion, wherein the flattened distal portion has a paddle geometric shape.

7. The system according to Claim 1, wherein the distal end of the guidewire is an atraumatic clot-circumventing configured distal end that is conformable in the lateral direction complementary to a contour of the inner wall of the target vessel when passed between the inner wall of the target vessel and the clot; and
when in a compressed state subject to application of an external mechanical force, a widest width in a lateral direction of the distal end of the guidewire is reduceable.

8. The system according to Claim 1, further comprising: a microcatheter comprising a proximal end and a distal end, wherein the guidewire is advanceable through the microcatheter; and
while in a non-compressed state not subject to application of an external mechanical force, the atraumatic clot-circumventing distal end of the guidewire having the widest width in the lateral direction greater than the inner diameter of the lumen, or greater than twice the inner diameter of the lumen.

9. An endovascular medical system for use with a clot located in a target vessel for managing one or more acute ischemic events, the system comprising:
a guidewire extending in a longitudinal direction from a proximal end to a distal end, wherein the guidewire comprises a plurality of temperature sensors, whereby at least one of temperature sensor is positioned proximal of the distal end and one or more of the other of the sensors are positioned on or adjacent the distal end, the one or more sensors configured for sensing temperature of the clot *in vivo* and identifying properties of the clot.

10. The system according to Claim 9, wherein the one or more sensors are configured to simultaneously measure temperature of a plurality of locations of the clot and target vessel.

11. The system according to Claim 9, wherein the sensors are selectively positioned at locations along the distal end to measure regions of the clot.

12. The system according to Claim 9, wherein the one or more sensors are each separated a predetermined distance at locations along the distal end to measure regions of the clot.

13. The system according to Claim 9, wherein the distal end of the guidewire comprises an expanded perimeter with an atraumatic clot-circumventing configured distal end in a delivery configuration, and wherein the one or more sensors are disposed about the expanded perimeter.

14. The system according to Claim 13, wherein the expanded perimeter has an elliptical, curved, or paddle geometric shape.

15. The system according to Claim 9, wherein the distal end of the guidewire comprises an expanded perimeter defined by a loop with a void therebetween in a delivery configuration, the loop comprising two elongate sections extended distally and joined at the distal end, and wherein the one or more sensors are disposed about the elongate sections.

16. The system according to Claim 15, wherein the loop and the void has an elliptical, curved, or paddle geometric shape.

17. The system according to Claim 9, wherein the distal end of the guidewire is conformable in the lateral direction complementary to a contour of the inner wall of the target vessel when passed between the inner wall of the target vessel and the clot; and
when in a compressed state subject to application of an external mechanical force, a widest width in a lateral direction of the distal end of the guidewire is reduceable.
